# EUROPEAN PATENT SPECIFICATION

(11) **EP 2 185 150 B1**
(45) Date of publication and mention of the grant of the patent: **14.03.2012**
(21) Application number: 08787273.5
(22) Date of filing: 15.08.2008
(51) Int. Cl.: A61K 31/33, A61P 25/00

(54) **USE OF TRANILAST AND DERIVATIVES THEREOF FOR THE THERAPY OF NEUROLOGICAL CONDITIONS**
VERWENDUNG VON TRANILAST UND SEINEN DERIVATEN ZUR BEHANDLUNG VON NEUROLOGISCHEN ZUSTÄNDEN
UTILISATION DE TRANILAST ET DE DÉRIVÉS DE CELUI-CI POUR LA THÉRAPIE D'ÉTATS NEUROLOGIQUES

(30) Priority: 17.08.2007 EP 07016189
(43) Date of publication of application: 19.05.2010
(73) Proprietor: Sygnis Bioscience GmbH & Co. KG, 69120 Heidelberg (DE)
(72) Inventor: SCHNEIDER, Armin, 69118 Heidelberg (DE); MORARU, Anja, 76889 Oberotterbach (DE); KRÜGER, Carola, 67434 Neustadt/Weinstrasse (DE); LAAGE, Rico, 69198 Schriesheim (DE); PITZER, Claudia, 69231 Rauenberg (DE)
(74) Representative: Lahrtz, Fritz
(86) International application number: PCT/EP2008/060745
(87) International publication number: WO 2009/024543

(56) References cited:
- WO-A-2004/071531
- WO-A-2006/053390
- WO-A-2006/076580
- WO-A-2006/122353

## Description

The present invention relates to the use of tranilast and derivatives thereof for the manufacture of a medicament for the treatment and/or prophylaxis of neurological and/or psychiatric conditions by enhancing or inducing neurogenesis and by protecting neurons from cell death. The invention furthermore relates to the use of tranilast and derivatives thereof for the in vitro differentiation of neuronal stem cells and the use of such pre-treated cells for stem cell therapy von neurological conditions.

Neurological conditions are medical conditions including diseases or disorder which affects the nervous system. The neurological condition may be accompanied by an pathologically impaired function of the nervous system, e.g., due to a disease or disorder, or may simply require an improvement of the neurological functions as achieved, e.g., by enhancing the cognitive ability in order to improve learning and memory.

Thes neurological conditions include amyotrophic lateral sclerosis (ALS), Huntington's disease and Glaucoma.

Most studies on cerebral ischemia and testing of pharmacological substances in vivo have only been concerned with the immediate effects of the drug or paradigm under investigation (i.e. infarct size 24 h after induction of the stroke). However, a more valid parameter of true efficacy of a particular substance is the long-term effect on functional recovery, which is also reflected in human stroke studies, where clinical scales (e.g., Scandinavian stroke scale, NIH scale, Barthel index) also reflect the ability to perform daily life activities. Recovery in the first few days after focal lesions may be due to resolution of edema or reperfusion of the ischemic penumbra. Much of the functional recovery after the acute phase is likely due to brain plasticity, with adjacent cortical areas of the brain taking over functions previously performed by the damaged regions (Chen et al., Neuroscience 2002; 111: 761-773). The two main mechanisms proposed to explain reorganization are unmasking of previously present but functionally inactive connections and growth of new connections such as collateral sprouting (Chen et al., Neuroscience 2002; 111: 761-773). Short term plastic changes are mediated by removing inhibition to excitatory synapses, which is likely due to reduced GABAergic inhibition (Kaas, Annu Rev Neurosci. 1991; 14: 137-167*;* Jones, Cereb Cortex. 1993; 3: 361-372). Plasticity changes that occur over a longer time involve mechanisms in addition to the unmasking of latent synapses such as long-term potentiation (LTP), which requires NMDA receptor activation and increased intracellular calcium concentration (Hess & Donoghue, Neurophysiol. 1994; 71: 2543-2547). Long term changes also involve axonal regeneration and sprouting with alterations in synapse shape, number, size and type (Kaas, Annu Rev Neurosci. 1991; 14: 137-167). Recent investigations show that the enhancement of neuroregenerative processes after cerebral ischemia improve the outcome (Fisher et al. Stroke 2006; 37: 1129-1136). There is a compelling need to develop cell and pharmacological therapeutic approaches to be administered beyond the hyperacute phase of stroke. Therefore, a successful future stroke therapy which is designed to reduce neurological deficits after stroke should approach besides revascularization at once also prevention of cell death, stimulation of neuroregeneration, and plasticity.

Cardiovascular diseases are the major cause of death in western industrialized nations. In the United States, there are approximately 1 million deaths each year with nearly 50% of them being sudden and occurring outside the hospital (*Zheng et al.,*

Amyotrophic lateral sclerosis (ALS; Lou-Gehrig's disease; Charcot's disease) is a neurodegenerative disorder with an annual incidence of 0.4 to 1.76 per 100.000 population (Adams et al., Principles of Neurology, 6.sup.th ed., New York, pp 1090-1095). It is the most common form of motor neuron disease with typical manifestations of generalized fasciculations, progressive atrophy and weakness of the skeletal muscles, spasticity and pyramidal tract signs, dysarthria, dysphagia, and dyspnea. The pathology consists principally in loss of nerve cells in the anterior horn of the spinal cord and motor nuclei of the lower brainstem, but can also include the first order motor neurons in the cortex. Pathogenesis of this devastating disease is still largely unknown, although the role of superoxide-dismutase (SOD1) mutants in familial cases has been worked out quite well, which invokes an oxidative stress hypothesis. So far, more than 90 mutations in the SOD1 protein have been described, that can cause ALS (Cleveland & Rothstein, Nat Rev Neurosci. 2001; 2: 806-819*).* Also, a role for neurofilaments in this disease was shown. Excitotoxicity, a mechanism evoked by an excess glutamate stimulation is also an important factor, exemplified by the beneficial role of Riluzole in human patients. Most convincingly shown in the SOD1 mutants, activation of caspases and apoptosis seems to be the common final pathway in ALS (Ishigaki et al., J Neurochem. 2002; 82: 576-584., Li et al., Science 2000 ; 288 : 335-339). Therefore, it seems that ALS also falls into the same general pathogenetic pattern that is also operative in other neurodegenerative diseases and stroke, i.e. glutamate involvement, oxidative stress, and programmed cell death.
Glaucoma is the number one cause of preventable blindness in the United States. Glaucoma is a group of conditions where the nerve of sight (the optic nerve) is damaged, usually as a result of increased pressure within the eye, but glaucoma can also occur with normal or even below-normal eye pressure. The lamina cribrosa (LC) region of the optic nerve head (ONH) is a major site of injury in glaucomatous optic neuropathy. It is a patchy loss of vision, which is permanent, but progress of the condition can be minimised if it is detected early enough and treatment is begun. However, if left untreated, glaucoma can eventually lead to blindness. Glaucoma is one of the most common eye disorders amongst older people. Worldwide, it is estimated that about 66.8 million people have visual impairment from glaucoma, with 6.7 million suffering from blindness.
There are a variety of different types of glaucoma. The most common forms are: Primary Open-Angle Glaucoma; Normal Tension Glaucoma; Angle-Closure Glaucoma; Acute Glaucoma; Pigmentary Glaucoma; Exfoliation Syndrome or Trauma-Related Glaucoma.
Glaucoma can be treated with eyedrops, pills, laser surgery, eye operations, or a combination of methods. The whole purpose of treatment is to prevent further loss of vision. This is imperative as loss of vision due to glaucoma is irreversible. Keeping the IOP under control is the key to preventing loss of vision from glaucoma. Recent developments emphasises the requirement of neuroprotection and neuroregeneration for the treatment of glaucoma. (Levin, Ophthalmol Clin North Am. 2005;18:585-596*, vii;* Schwartz et al., J Glaucoma. 1996; 5: 427-432).

Parkinson's disease is the most frequent movement disorder, with approximately 1 million patients in North America; about 1 percent of the population over the age of 65 years is affected. The core symptoms of the disease are rigor, tremor and akinesia (Adams et al., Principles of Neurology, 6.sup.th ed., New York, pp 1090-1095). The etiology of Parkinson's disease is not known. Nevertheless, a significant body of biochemical data from human brain autopsy studies and from animal models points to an ongoing process of oxidative stress in the substantia nigra, which could initiate dopaminergic neurodegeneration. Oxidative stress, as induced by the neurotoxins 6-hydroxydopamine and MPTP (N-methyl-4-phenyl-1,2,3,6-tetrahydropyridine), has been used in animal models to investigate the process of neurodegeneration. Although a symptomatic therapy exists (e.g. L-DOPA plus a decarboxylase inhibitor; bromocriptine, pergolide as dopamin agonists; and anticholinergic agents such as trihexyphenidyl (artane)), there is a clear need for a causative therapy, e.g. a neuroprotective and/or neuroregenerative therapy, that really halts the disease progress or even reverses it. These animal models have been used to test the efficacy of radical scavengers, iron chelators, dopamine agonists, nitric oxide synthase inhibitors and certain calcium channel antagonists. Apoptotic mechanisms are clearly operative in the animal models as well as in the patient (Mochizuki et al., Proc. Natl. Acad. Sci. USA 2001; 98:10918-10923*;* Xu et al., Nat. Med. 2002; 8:600-606*;* Viswanath et al., J. Neurosci. 2001; 21:9519-9528*;* Hartmann et al., Neurology 2002; 58:308-310). This pathophysiology with involvement of oxidative stress and apoptosis also places Parkinson's disease amongst the other neurodegenerative disorders and stroke.
Pathologically Parkinson's disease is defined as a neurodegenerative disorder characterized chiefly by depigmentation of the substantia nigra and by the presence of Lewy bodies. These criteria, however, are too restrictive and simple, and they do not take into account the heterogeneous clinical and pathologic presentation of Parkinson's disease and the overlap with other parkinsonian disorders, each with presumably distinct etiology. In the absence of a specific biologic marker for Parkinson's disease, the differentiation of Parkinson's disease from other parkinsonian disorders rests on clinicopathologic criteria (Dauer & Przedborski, Neuron 2003; 39:889-909).

Alzheimer's disease (AD) is a neurodegenerative disease characterized by progressive cognitive deterioration together with declining activities of daily living and neuropsychiatric symptoms or behavioral changes. It is the most common type of dementia. The most striking early symptom is loss of memory, which usually manifests as minor forgetfulness that becomes steadily more pronounced with illness progression, with relative preservation of older memories. The pathological process consists principally of neuronal loss or atrophy, principally in the temporoparietal cortex, but also in the frontal cortex, together with an inflammatory response to the deposition of amyloid plaques and neurofibrillary tangles. The ultimate cause of the disease is unknown. Three major competing hypotheses exist to explain the cause of the disease. The oldest, on which most currently available drug therapies are based, is known as the "cholinergic hypothesis" and suggests that AD is due to reduced biosynthesis of the neurotransmitter acetylcholine. Levels of acetylcholine are reduced in brain tissue of AD patients, whereas glutamate levels are usually elevated. The medications that treat acetylcholine deficiency have served to only treat symptoms of the disease and have neither halted nor reversed it (Walker & Rosen, Age Ageing 2006; 35:332-335). Research after 2000 includes hypotheses centred on the effects of the misfolded and aggregated proteins, amyloid beta and tau. The two positions differ with one stating that the tau protein abnormalities initiate the disease cascade, while the other believes that beta amyloid deposits are the causative factor in the disease (Mudher & Lovestone, Trends Neurosci. 2002; 25:22-26).
There is currently no cure for AD. Currently available medications offer relatively small symptomatic benefit for some patients but do not slow disease progression. It helps a little for the memory (Lyketsos et al., Am J Geriatr Psychiatry. 2006; 14:561-572). Acetylcholinesterase (AChE) inhibition was thought to be important because there is a reduction in activity of the cholinergic neurons. Acetylcholinesterase-inhibitors seemed to modestly moderate symptoms but do not alter the course of the underlying dementing process. There is significant doubt as to the effectiveness of cholinesterase inhibitors. One of the natural extracts that has been examined in AD is Ginkgo (*Ginkgo biloba*). A large, randomized clinical study in the US is underway and examining the effect of Ginkgo to prevent dementia (DeKosky et al., Contemp Clin Trials 2006, 27: 238-253). Recent evidence of the involvement of glutamatergic neuronal excitotoxicity causes AD led to the development and introduction of memantine, a novel NMDA receptor antagonist, which has been shown to be moderately clinically efficacious (Areosa-Sastre et al., Cochrane Database Syst Rev. 2004; 18: CD003154*).* Recent investigations on the processes of neurogenesis and the observation of widespread existence of endogenous neural progenitors offers hope that the potential of these cells may be harnessed to repair neurodegenerative diseases such as AD (Elder et al., Mt Sinai J Med. 2006; 73:931-940*;* Brinton & Wang, Curr Alzheimer Res. 2006; 3:185-190*;* Kelleher-Andersson, Curr Alzheimer Res. 2006; 3: 55-62; Greenberg & Jin, Curr Alzheimer Res. 2006; 3: 25-8).

One group of Neurodegenerative disorders is characterized by an expansion of trinucleotides. Those neurodegenerative trinucleotide repeat disorders are chronic and progressive characterised by selective and symmetric loss of neurons in motor, sensory, or cognitive systems. Symptoms are often ataxia, dementia or motor dysfunction. The best known trinucleotide repeat disorder is Huntington's disease, others are Spinal and bulbar muscular atrophy (Kennedy's disease), Autosomal dominant spinocerebellar ataxia's: Type 1 SCA1, Type 2 SCA2, Type 3 (Machado-Joseph disease) SCA3/MJD, Type 6 SCA6, Type 7 SCA7, Type 8 SCA8, Friedreich's Ataxia and Dentatorubral pallidoluysian atrophy DRPLA/Haw-River syndrome. (Hardy & Gwinn-Hardy, Science 1998; 282: 1075-1079*;* Martin, N Engl J Med. 1999; 340: 1970-1980*;* Schols et al., Ann Neurol. 1997; 42: 924-932)*.*
Huntington's disease (HD) is an autosomal dominant, inherited, neuropsychiatric disease which gives rise to progressive motor, cognitive and behavioural symptoms. The course of HD is characterized by jerking uncontrollable movement of the limbs, trunk, and face (chorea); progressive loss of mental abilities; and the development of psychiatric problems. HD progresses without remission over 10 to 25 years and usually appears in middle age (30-50 years). Juvenile HD (also called Westphal variant or akinetic-rigid HD) develops before the age of 20, progresses rapidly, and produces muscle rigidity in which the patient moves little, if at all (akinesia). It is estimated that one in every 10,000 persons - nearly 30,000 in the United States - have HD. Juvenile HD occurs in approximately 16% of all cases. Its core pathology involves degeneration of the basal ganglia, in particular, the caudate and putamen, and is caused by an unstable expansion of the trinucleotide CAG, coding for glutamine, in a single autosomal gene IT-15 on chromosome 4, coding for a mutated form of the protein, Huntingtin. How the mutation of gene IT-15 alters the function of the protein is not well understood.
Treatment of HD focuses on reducing symptoms, preventing complications, and providing support and assistance to the patient. There are several substances available today for the treatment of chorea. Other neurological symptoms, such as dystonia, can be treated, but treatment is associated with a high risk of adverse events. Psychiatric symptoms, on the other hand, are often amenable to treatment and relief of these symptoms may provide significant improvement in quality of life.
(Bonelli & Hofmann, Expert Opin Pharmacother. 2004; 5: 767-776). Most drugs used to treat the symptoms of HD have side effects such as fatigue, restlessness, or hyperexcitability. Cystamine (=Decarboxycystine) alleviates tremors and prolongs life in mice with the gene mutation for HD. The drug appears to work by increasing the activity of proteins that protect nerve cells, or neurons, from degeneration. The study suggests that a similar treatment or a neuroregenerative treatment may one day be useful in humans with HD and related disorders. (Karpuj et al., Nat Med. 2002; 8: 143-149).

The lysosomal storage diseases (LSD) are a group of about 40 different diseases, each characterised by a specific lysosomal enzyme deficiency in a variety of tissues. They occur in total in about 1 in 5,000 live births and display considerable clinical and biochemical heterogeneity. The majority are inherited as autosomal recessive conditions although two (Hunter disease and Fabry disease) are X-linked. They include Tay-Sachs disease, a gangliosidosis, and Gaucher's and Niemann-Pick's diseases, which are lipid storage disorders. Most of these diseases affect the brain and are fatal. (Brooks et al., Proc Natl Acad Sci USA 2002; 99: 6216-6221).
There has been limited success in only treating the symptoms of these diseases. One way is to replace the enzyme to put a normal gene into the body that can make the enzyme by bone marrow or stem cell transplant or gene therapy. Bone marrow transplantation (BMT) has been successful in several LSDs and allowed long term survival with less severe symptoms. Enzyme replacement therapy (ERT) has been available for patients with Gaucher disease for over 10 years and has provided enormous benefit. However, in many incidents the neurons are unable to take up the large enzyme efficiently even when it is placed next to the cell, so the treatment is still ineffective. It is expected that the stimulation of neuroregeneration and the protection of neurons from apoptosis can at least alleviate the neuronal symptoms and the progress of such lysosomal storage diseases or have a prophylactic effect.

Multiple sclerosis (MS) is the prototype inflammatory autoimmune disorder of the central nervous system and, with a lifetime risk of one in 400, potentially the most common cause of neurological disability in young adults. Worldwide, there are about 2-5 million patients suffering from this disease (Compston & Coles, Lancet 2002; 359: 1221-1231). As with all complex traits, the disorder results from interplay between as yet unidentified environmental factors and susceptibility genes. Together, these factors trigger a cascade of events, involving engagement of the immune system, acute inflammatory injury of axons and glia, recovery of function and structural repair, post-inflammatory gliosis, and neurodegeneration. The sequential involvement of these processes underlies the clinical course characterized by episodes with recovery, episodes leaving persistent deficits, and secondary progression. The aim of treatment is to reduce the frequency, and limit the lasting effects of relapses, relieve symptoms, prevent disability arising from disease progression, and promote tissue repair.
Recently, neuroprotection has been proclaimed an important goal for MS therapy. The basis for widening the scope of neuroprotection is evidence that neuronal and axonal injury are key features of MS lesions. Axon loss most likely determines the persistent neurological deficit in progressive MS. Recent studies pointed out that axon damage occurs early in the disease and during lesion development. Two different phases of axon degeneration were characterized, the first occurring during active myelin breakdown and the second in chronic demyelinated plaques in which the naked axon seems more susceptible to further damage. In contrast with degenerative and ischaemic central nervous system injury, however, neurodegeneration in MS appears to be caused by an inflammatory, presumably autoimmune, process. The challenge for neuroprotection in MS is therefore greater than in degenerative and ischaemic disorders, because MS requires the combination of neuroprotective therapy and effective immunomodulation. In order to reverse already acquired neurodegeneration, a successful future MS therapy which is designed to reduce neurological deficits should approach besides immunomodulation also stimulation of neurogenesis. The exact mechanisms and effector molecules of axonal degeneration, however, are not yet defined, and an axon-protective therapy has not yet been established. (Bruck & Stadelmann, Neurol Sci. 2003; 24: S265-S267*;* Hohlfeld, Int MS J 2003; 10: 103-105).

Spinal cord injury (SCI) occurs when a traumatic event results in damage to cells within the spinal cord or severs the nerve tracts that relay signals up and down the spinal cord. The most common types of SCI include contusion (bruising of the spinal cord) and compression (caused by pressure on the spinal cord). Other types of injuries include lacerations (severing or tearing of some nerve fibers, such as damage caused by a gun shot wound), and central cord syndrome (specific damage to the corticospinal tracts of the cervical region of the spinal cord). Severe SCI often causes paralysis (loss of control over voluntary movement and muscles of the body) and loss of sensation and reflex function below the point of injury, including autonomic activity such as breathing and other activities such as bowel and bladder control. Other symptoms such as pain or sensitivity to stimuli, muscle spasms, and sexual dysfunction may develop over time. SCI patients are also prone to develop secondary medical problems, such as bladder infections, lung infections, and bed sores. While recent advances in emergency care and rehabilitation allow many SCI patients to survive, methods for reducing the extent of injury and for restoring function are still limited. Immediate treatment for acute SCI includes techniques to relieve cord compression, prompt (within 8 hours of the injury) drug therapy with corticosteroids such as methylprednisolone to minimize cell damage, and stabilization of the vertebrae of the spine to prevent further injury. The types of disability associated with SCI vary greatly depending on the severity of the injury, the segment of the spinal cord at which the injury occurs, and which nerve fibers are damaged. It has long been believed that the adult mammalian central nervous system does not regenerate after SCI. However, cell replacement has been observed recently as an extensive natural compensatory response to injury in the primate spinal cord that contributes to neural repair and is a potential target for therapeutic enhancement (Yang et al., J Neurosci. 2006; 26: 2157-2166). Advances in the field of stem cell biology, including the identification neural stem cells, has provided new insight for the development of novel therapeutic strategies aimed at inducing regeneration in the damaged central nervous system , i.e. the activation of endogenous these neural stem cells (Okano, Ernst Schering Res Found Workshop. 2006; 60:215-228).

Dementia is the progressive decline in cognitive function due to damage or disease in the brain beyond what might be expected from normal aging. Particularly affected areas may be memory, attention, language, and problem solving. Especially in the later stages of the condition, affected persons may be disoriented in time, in place, and in person. For the majority of dementia subtypes there is no cure to this illness, although scientists are progressing in making a type of medication that will slow down the process. Cholinesterase inhibitors are often used early in the disease course. Cognitive and behavioral interventions may also be appropriate. Memantine within the class known as N-methyl-D-aspartate (NMDA) blockers has been approved by the FDA for the treatment of moderate-to-severe dementia. Impaired neurogenesis plays an important role for the decline of cognitive function, e.g. memory, during dementia. It has been shown that recovery of neuronal cell production was associated with the ability to acquire trace memories. The results indicate that newly generated neurons in the adult participate in the formation of a hippocampal-dependent memory (Shors et al., Nature 2001; 410:372-376). Neurosciences have shown that there is a biology of cognition and that neurogenesis and apoptosis are permanently in confrontation in the brain. The present targets of pharmacology are directed against the decline of the neurone and of cognitive performance (Allain et al., Psychol Neuropsychiatr Vieil. 2003; 1:151-156).

Schizophrenia is one of the most common mental illnesses. About 1 of every 100 people (1% of the population) is affected by schizophrenia. This disorder is found throughout the world and in all races and cultures. Schizophrenia affects men and women in equal numbers, although on average, men appear to develop schizophrenia earlier than women. Generally, men show the first signs of schizophrenia in their mid 20s and women show the first signs in their late 20s. Schizophrenia has a tremendous cost to society, estimated at $32.5 billion per year in the US. Schizophrenia is characterized by several of the following symptoms: delusions, hallucinations, disorganized thinking and speech, negative symptoms (social withdrawal, absence of emotion and expression, reduced energy, motivation and activity), catatonia. The main therapy for schizophrenia is based on neuroleptics, such as chlorpromazine, haloperidol, olanzapine, clozapine, thioridazine, and others. However, neuroleptic treatment often does not reduce all of the symptoms of schizophrenia. Moreover, antipsychotic treatment can have severe side effects, such as tardive dyskinesias. The etiology of schizophrenia is not clear, although there seems to be a strong genetic influence. Recently, it has become clear that schizophrenia has at least some aspects of a neurodegenerative disease. In particular, MR studies have revealed rapid cortical grey matter loss in schizophrenic patients (Thompson et al., Proc Natl Acad Sci USA 2001; 98: 11650-11655*;* Cannon et al., Proc Natl Acad Sci USA 2002; 99: 3228-3233). Therefore, treatment of schizophrenics with neuroprotective and/or neuroregenerative medication is warranted.

Peripheral neuropathy is a pain initiated or caused by a primary lesion or dysfunction of the nervous system. Many classification systems exist but typically it is divided into central (i.e. thalamic, post-stroke pain) and peripheral deafferent pain (i.e. meralgia paresthetica). Neuropathies may affect just one nerve (mononeuropathy) or several nerves (polyneuropathy). They are allodynia, hyperalgesia, and dysesthesias. Common symptoms include burning, stabbing, electric shock, or deep aching sensations. The causes of neuralgia include diabetic neuropathy, trigeminal neuralgia, complex regional pain syndrome and post-herpetic neuralgia, uremia, AIDS, or nutritional deficiencies. Other causes include mechanical pressure such as compression or entrapment, direct trauma, penetrating injuries, contusions, fracture or dislocated bones; pressure involving the superficial nerves (ulna, radial, or peroneal) which can result from prolonged use of crutches or staying in one position for too long, or from a tumor; intraneural hemorrhage; exposure to cold or radiation or, rarely, certain medicines or toxic substances; and vascular or collagen disorders such as atherosclerosis, systemic lupus erythematosus, scleroderma, sarcoidosis, rheumatoid arthritis, and polyarteritis nodosa. A common example of entrapment neuropathy is carpal tunnel syndrome, which has become more common because of the increasing use of computers. Although the causes of peripheral neuropathy are diverse, they produce common symptoms including weakness, numbness, paresthesia (abnormal sensations such as burning, tickling, pricking or tingling) and pain in the arms, hands, legs and/or feet. A large number of cases are of unknown cause.
Treating the underlying condition may relieve some cases of peripheral neuropathy. In other cases, treatment may focus on managing pain. Therapy for peripheral neuropathy differs depending on the cause. For example, therapy for peripheral neuropathy caused by diabetes involves control of the diabetes. In cases where a tumor or ruptured disc is the cause, therapy may involve surgery to remove the tumor or to repair the ruptured disc. In entrapment or compression neuropathy treatment may consist of splinting or surgical decompression of the ulnar or median nerves. Peroneal and radial compression neuropathies may require avoidance of pressure. Physical therapy and/or splints may be useful in preventing contractures. Peripheral nerves have a remarkable ability to regenerate themselves, and new treatments using nerve growth factors or gene therapy may offer even better chances for recovery in the future. Therefore, the enhancement of this regeneration of the peripheral nerves by neuroregenerative medication is warranted.

In humans there is a need for ways to increase cognitive capacities, and boost intelligence. "Intelligence" in modern understanding is not limited to purely logical or semantic capabilities. For example, the theory of multiple intelligences by Howard Gardner evaluates intelligence from evolutionary and anthropological perspectives and yields a broader view that includes athletic, musical, artistic, and empathetic capacities as well as the linguistic/logical abilities that are more commonly associated with intelligence and measured by IQ tests. This broader sense of intelligence also extends into the area of creativity. In addition, there is a non-pathological condition known in the human as ARML (age-related memory loss) or MCI (mild cognitive impairment) or ARCD (age-related cognitive decline) that usually commences at about age 40, and is different from early signs of Alzheimer's disease.
There is a physiological loss of nerve cells throughout adulthood, estimated to as many as 100,000 neurons a day. Throughout adulthood, there is a gradual reduction in the weight and volume of the brain. This decline is about 2% per decade. Contrary to previously held beliefs, the decline does not accelerate after the age of 50, but continues at about the same pace from early adulthood on. The accumulative effects of this are generally not noticed until older age.
While the brain does shrink in size, it does not do so uniformly. Certain structures are more prone to shrinkage. For example, the hippocampus and the frontal lobes, two structures involved in memory, often become smaller. This is partly due to a loss of neurons and partly due to the atrophy of some neurons. Many other brain structures suffer no loss in size. The slowing of mental processing may be caused by the deterioration of neurons, whether they are lost, shrink, or lose connections. This depletion of fully functioning neurons makes it necessary to recruit additional networks of neurons to manage mental tasks that would otherwise be simple or automatic. Thus, the process is slowed down.
A portion of the frontal lobe, called the prefrontal cortex, is involved in monitoring and controlling thoughts and actions. The atrophy that occurs in this brain region may account for the word finding difficulties many older adults experience. It may also account for forgetting where the car keys were put or general absentmindedness. The shrinkage of both the frontal lobe and the hippocampus are thought to be responsible for memory difficulties. Therefore, there also remains a need for improving or enhancing the cognitive ability of an individual. This may be achieved by the enhancement of neuroprotective and/or neuroregenerative processes of the brain.

In view of the above, there is a need for treating neurological and/or psychiatric conditions, such as neurological diseases that relate to the need of enhanced plasticity and functional recovery, or of suppressed cell-death in the nervous system by providing neuroprotection to the neural cells involved or by inducing neurogenesis to recover from neuronal loss. In particular, there is a need for therapeutic means facilitating or inducing neurogenesis in order to ameliorate or recover from neuronal loss accompanying the aforementioned neurological and/or psychiatric conditions.

Accordingly, the technical problem underlying the present invention may be seen as the provision of means and methods for facilitating or inducing neurogenesis and, thereby, for treating, ameliorating and/or preventing neurological and/or psychiatric conditions caused by neurodegeneration or impaired or insufficient neurogenesis. The technical problem is solved by the embodiments characterized in the claims and herein below.

Accordingly, the present invention relates to the use of a compound for the preparation of a pharmaceutical composition for treating and/or preventing a neuronal condition by enhancing or inducing neurogenesis, the compound having the following general formula (I) 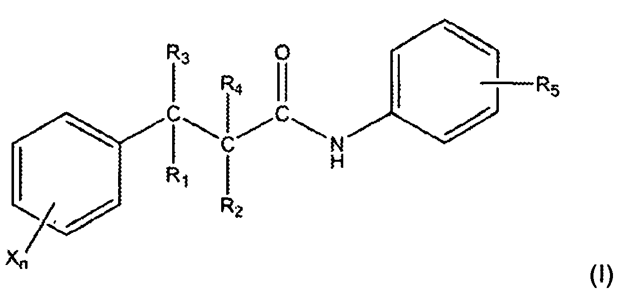 wherein
- R₁ and R₂: are independently a hydrogen atom or an alkyl group having from 1 to 6 carbon atoms;
- R₃ and R₄: are each hydrogen atoms, or R₃ and R₄ together form a second carbon-to-carbon bond resulting in a cis- or trans-alkene moiety;
- R₅ is a: group C(O)OR₅ₐ, an alkyl group having from 1 to 6 carbon atoms, or an alkoxy group having from 1 to 6 carbon atoms, whereby R₅ₐ is hydrogen or an alkyl group having from 1 to 6 carbon atoms;
- n=0,: 1,2, or 3; and
- each: X is independently a hydroxyl group, a halogen atom, a nitro group, an alkyl group having from 1 to 6 carbon atoms, or an alkoxy group having from 1 to 6 carbon atoms;
- optionally,: two groups X are joined to form an alkylene group having from 1 to 6 carbon atoms, wherein the alkylene group is optionally interrupted by one or more oxygen atoms.

Preferred compounds to be used in accordance with the present invention have the general formula (II) 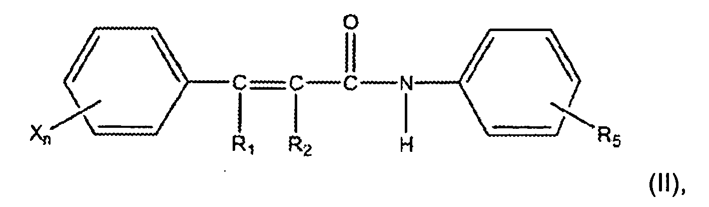 in cis- or trans-form, preferably in trans form, wherein X, n, R₁, R₂, and R₅ have the meaning as indicated above.

Preferably, R₁ and R₂ are hydrogen atoms.

Preferably, any of the alkoxy or alkyl groups mentioned above have from 1 to 4 carbon atoms. More preferably, said alkoxy groups are methoxy or ethoxy groups and said alkyl groups are methyl and ethyl groups.

The compounds of general formulas I and II may represent tranilast and derivatives thereof. A large number of compounds within formulas I and II have been synthesised and tested within the context of the treating allergies, as described in US 3,940,422. Accordingly, the compounds for the use according to the present invention may be prepared according to US 3,940,422 optionally in combination with further methods known in the art, included functional group protection, activation and transformation methods.

More preferably, the compound to be used in accordance with the present invention is tranilast, (N-(3',4'-methylenedioxycinnamoyl)-anthranilic acid, 2-(3',4'-dimethoxycinnamoylamino)-toluene, N-cinnamoyl-anthranilic acid, 2-(3',4'-dimethoxycinnamoylamino)-anisole, or 3-(4'-chlorocinnamoylamino)-anisole. Most preferably, the compound to be used in accordance with the present invention is tranilast.

Tranilast is also known as N-(3,4-dimethoxycinnamoyl)-anthranilic acid or 2- ((3 -(3,4-dimethoxyphenyl)-I-oxo-2-propenyl)amino)benzoic acid, or Rizaben® and is represented by the formula III

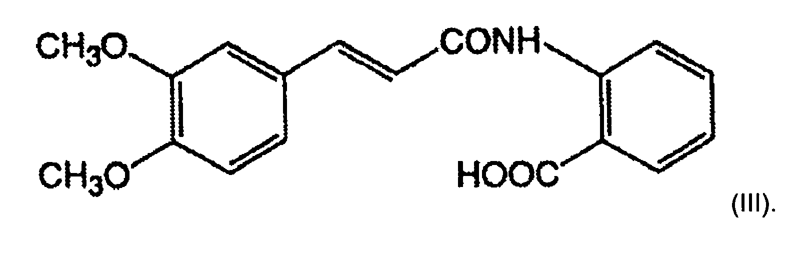

One embodiment of the present invention is a method of treating a patient being in need of neurogenesis comprising administering in a therapeutically effective amount a compound as defined above.

The aforementioned compounds to be applied in the uses and methods of the present invention, preferably, retain at least 10%, more preferably 50% of the neuroregenerative activity of tranilast as measured by the in vitro assays described herein.

The compounds to be used according to the present invention also include salts, hydrates, pro-drugs, isomers, tautomers, and/or metabolites.

The term "alkyl" used either alone or in a compound word denotes straight chain, branched or cyclic alkyl. Examples of straight chain and branched alkyl include methyl, ethyl, propyl, isopropyl, n-butyl, isobutyl, sec-butyl, tert-butyl, n-pentyl, 2-methylbutyl, n-hexyl, 2-methylpentyl, 3-methylpentyl, and the like. Examples of cyclic alkyl include cyclopropyl, cyclobutyl, cyclopentyl, cyclohexyl and the like. The alkyl may optionally be substituted by any non-deleterious substituent. The alkyl chains may have 1 to 6 carbon atoms, but have preferably 1 to 4 carbon atoms (methyl, ethyl, propyl, isopropyl, n-butyl, isobutyl, sec-butyl, tert-butyl).

The term "alkylene" means a group -(CH₂)ₘ-, wherein m denotes the number of carbon atoms, which is from 1 to 6, preferably from 1 to 4, more preferred 1 or 2. The term "optionally interrupted by one or more oxygen atoms" means that an oxygen may occur at one end or at each end of the alkylene group. Examples are -O-CH₂-O-, -O-CH₂-CH₂-O-, -O-CH₂-CH₂-CH₂-O-, -O-CH₂-CH₂.

The term "alkoxy" used either alone or in compound words denotes straight chain or branched alkoxy. Examples of alkoxy include methoxy, ethoxy, n- propyloxy, isopropyloxy and the various butyloxy isomers.

Halogen refers to chloro, fluoro, iodo and bromo.

The term "pro-drug" is used herein in its broadest sense to include those compounds which are converted in vivo to compounds of formula I or preferably of formula II, e.g., organic acid esters or ethers.

The term "tautomer" is used herein in its broadest sense to include compounds of formula I or preferably of formula II which are capable of existing in a state of equilibrium between two isomeric forms. Such compounds may differ in the bond connecting two atoms or groups and the position of these atoms or groups in the compound.

The term "isomer" is used herein in its broadest sense and includes structural, geometric and stereo isomers. As compounds of formula I or formula II may have one or more chiral centres, it is capable of existing in enantiomeric forms.

Tranilast exhibits anti-allergenic action (Azuma et al., Br J Pharmacol. 1976; 58:483-488*;* US 3,940,422*).* The anti-allergenic action is mediated by the inhibition of mast cell degranulation which leads to a reduced histamine release (Zampini et al., Int J Immunopharmacol. 1983; 5:431-435). A large number of compounds within formulas I and II have been synthesised and tested within the context of the treatment of allergies, as described in US 3,940,422*.* A more comprehensive group of derivatives of tranilast has been described later on (*WO 2005*/*110392; WO 2006*/*053390; WO 2006*/*076580*).

Due to its anti-allergenic action tranilast is orally administered as a therapeutical agent in a dosage of approximately 300 mg daily for the clinical treatment of allergic diseases such as bronchial asthma, atopic dermatitis and the like. However, tranilast is so insoluble in water that initially only pharmaceutical compositions for oral administration have been employed in the treatment of allergic diseases. The need for pharmaceutical compositions in a form for topical application, such as eye drops and nasal drops led to the development of aqueous formulations comprising tranilast (US 5, 356, 620*).*

Tranilast also have been found to inhibit dose dependently the aggregation of stimulated platelets suggesting that it may be used also as an anti-platelet agent (Iwasa et al., Eur J Pharmacol. 1986; 120:231-234).

Additionally, tranilast has been reported to inhibit fibroblast proliferation and collagen accumulation (Isaji et al. Biochem Pharmacol 1987; 36:469-474). Experimental results suggest that tranilast inhibits collagen synthesis of normal fibroblasts and of fibroblasts from keloid and hypertrophic scar tissue through suppressing the release of TGF-beta-1 from the fibroblasts themselves, attesting to its therapeutic potential as an anti-fibrotic drug (Suzawa et al., Japan J Pharmacol 1992; 60:85-90*;* Suzawa et al., Japan J Pharmacol 1992; 60:91-96*;* Yamada et al., J Biochem 1994; 116:892-897). Therefore, tranilast in a dosage of 300 mg to 1000 mg daily over a period of 3 months has been found to be effective in the prevention of restenosis after percutaneous transluminal coronary angioplasty (EP 588518). It is supposed that tranilast additionally to its anti-proliferative effect on fibroblasts also attenuates the proinflammatory activity of monocytes, adding to its potential efficacy as a therapeutic agent in restenosis (Capper et al., J Pharmacol Exp Ther. 2000; 295:1061-1069). Further, tranilast mediated suppression of TGF-beta-1 has also been observed in glioma cells accompanied with an inhibition of migration, chemotactic responses, and invasiveness which led to the conclusion that tranilast might be an useful therapeutic agent for the treatment of human malignant glioma (Platten et al., Int J Cancer. 2001; 93:53-61).

The suppressive effect on fibrosis may involve the inhibition of activated macrophages known to release nitric oxide (Suzawa et al., Japan J Pharmacol 1992; 60:85-90*).* On the other hand, there are lines of evidence that tranilast may be a positive regulator of inducible nitric oxide synthase (iNOS) (Hishikawa et al., J Cardiovasc Pharmacol. 1996; 28:200-207). Recently, it has been reported that after induction with interferon-gamma or bacterial endotoxin lipopolysaccharide the expression and activity of microglial iNOS is suppressed by tranilast. Therefore, it has been supposed to use tranilast as a protective agent against increased NO production (Platten et al., Br J Pharmacol. 2001; 134:1279-1284*;* Platten et al., Biochem Pharmacol. 2003; 66:1263-1270).

Tranilast also has been reported to inhibit proliferation of human dermal microvascular endothelial cells and thereby to inhibit angiogenesis. Therefore, it is supposed to be beneficial for the improvement of angiogenic diseases such as proliferative diabetic retinopathy, age-related macular degeneration, tumour invasion and rheumatoid arthritis (Isaji et al., Br J Pharmacol. 1997; 122:1061-1066).

Recently, it has been described that tranilast and derivatives thereof are potent inhibitors of the FMS-like tyrosine kinase 3 (FLT3). Although the normal role of FLT3 has not yet been completely understood, it is thought to be involved in the regulation of survival and proliferation of haematopoietic progenitor cells. Abnormal expression and/or activity of FLT3 has been associated with several haematopoietic disorders, such as acute myelogenous leukaemia. Therefore, tranilast and or derivatives thereof are supposed to be beneficial for the treatment of haematological malignancy, such as leukaemia (*WO 2005*/*110392*).

Furthermore, tranilast can be considered as derivative of tryptophan catabolites. The catabolism of the amino acid tryptophan by indoleamine-2,3-dioxygenase leads to 3-hydroxykynurenic acid and subsequently to 3-Hydroxyanthranilic acid which in turn is a derivative of the anthralinic acid moiety of tranilast. Finally, 3-Hydroxyanthranilic acid is further processed to picolinic acid and to quinolinic acid. Recently, it has been observed that these tryptophan catabolites as well as tranilast as a derivative thereof have the ability in common to suppress certain B- and T- cell proliferation (Platten et al., Science 2005; 310:850-855*; WO 2006*/*053390; WO 2006*/*076580*). Accordingly, as one embodiment of the present invention it has been found that said tryptophan catabolites as well as derivatives thereof can be used according to the present invention additionally to the compounds of formulas I and II.

3-(4'-chlorocinnamoylamino)-anisole (also SB-366791 or N-(3-methoxyphenyl)-4-chlorocinnamide) is a selective and potent inhibitor of the vanilloid receptor subtype TRPV1 and therefore potentially useful for the suppression of chronic pain or inflammatory processes (Varga et al., Neurosci Lett. 2005; 385:137-142*;* Gunthrope et al., Neuropharmacology 2004; 46:133-146*).*

Taken together tranilast has been demonstrated to be an anti-allergenic, anti-fibrotic and anti-inflammatory agent. However, it has been unknown that tranilast or derivatives thereof enhance neurogenesis and act protective on neurons. According to the present invention tranilast unexpectedly has been found to induce or enhance neurogenesis and, thereby, can be used for treating neuronal conditions which benefit from enhanced neurogenesis.

The term "neurogenesis" as used herein refers, in principle, to the formation of neurons from stem cells. Recently, the importance of forming new nerve cells (neurogenesis) for treating neurological disease has been recognized. Unlike many other tissues, the mature brain has limited regenerative capacity, and its unusual degree of cellular specialization restricts the extent to which residual healthy tissue can assume the function of damaged brain. However, cerebral neurons are derived from precursor cells that persist in the adult brain, so stimulation of endogenous neural precursors in the adult brain could have therapeutic potential.
Neurogenesis bases on the differentiation of neuronal stem cells to new neurons. It occurs in discrete regions of the adult brain, including the rostral subventricular zone (SVZ) of the lateral ventricles and the subgranular zone (SGZ) of the dentate gyrus (DG). Neurogenesis occurs in the adult animal especially after a particular neurological paradigm - e.g. cerebral ischemia (Jin et al., Proc. Natl. Acad. Sci. USA 2001; 98: 4710-4715 *;* Jiang et al., Stroke 2001; 32: 1201-1207 *;* Kee et al., Exp. Brain. Res. 2001; 136: 313-320 *;* Perfilieva et al., J. Cereb. Blood Flow Metab. 2001; 21: 211-217). Neurogenesis has also been demonstrated in humans (Eriksson et al., Nat Med. 1998; 4: 1313-1317), and indeed leads to functional neurons (van Praag et al., Nature 2002; 415: 1030-1034*).* In particular, the subgranular zone of the dentate gyrus, and the hilus has the potential to generate new neurons from adult neuronal stem cells (Gage et al., J Neurobiol 1998; 36: 249-266).

Adult neuronal stem cells do not possess the ability to differentiate into all cell types of the organism (totipotent) as embryonal stem cells do, but they have the potential to differentiate to the various cell types of the brain tissue (pluripotent). During the differentiation they undergo essential morphological and functional changes (van Praag et al., Nature 2002; 415: 1030-1034). The regeneration of neuronal cells (neuroregeneration) depends on the occurrence of neurogenesis and allows for the recovery of neurodegenerative processes. Since the necessity of regeneration of neuronal tissue is similar in both acute and chronic neurodegenerative diseases, compounds such as tranilast and derivatives thereof which possess the ability to induce or enhance neurogenesis are generally suitable for treating these diseases.

The term "neuroprotection" means processes within the nervous system which protect neurons from apoptosis or degeneration, for example following a brain injury or as a result of chronic neurodegenerative diseases. The goal of neuroprotection is to limit neuronal dysfunction/death after CNS injury and attempt to maintain the highest possible integrity of cellular interactions in the brain resulting in an undisturbed neural function. There is a wide range of neuroprotection products under investigation (e.g. free radical scavengers, Apoptosis inhibitors, neurotrophic factors, metal ion chelators, and anti-excitotoxic agents).

Advantageously, neuroregenerative acting compounds, such as tranilast and derivatives thereof, allow for the treating of a neurological condition even at a late stage after progressive neuronal loss. Such compounds enable to reverse neuronal loss which have had already occurred. By contrast a compound which solely acts neuroprotective can be used only to stop or attenuate the ongoing process of neuronal dieing.
Specifically, it has been unexpectedly found that the compounds referred to in accordance with the present invention have a neuroprotective and neuroregenerative effect. Therefore, these compounds can be used alone or in combination for the treatment of neurological conditions or diseases where there is a need of neuroregeneration, particularly, where there is a need of neuroprotection and neuroregeneration.

Thus, contemplated by the present invention is also a method of treating a patient with a neurological condition comprising administering in a therapeutically effective amount a compound as defined above to a patient suffering from the said neurological condition. Preferred neurological conditions or diseases to be treated are those referred to elsewhere in this specification explicitly.

It has been shown that the compounds according to the present invention have the ability to enhance neurogenesis, and therefore e.g. improve behavioural outcome after an ischemic lesion. Neurogenesis is one mechanism that can lead to increased plasticity of neural networks, and can replace gradual loss of neurons. Therefore, one embodiment of the present invention is to provide enhancement, improvement or an increase in cognitive ability to an individual suffering from, displaying, and/or believed to some level of cognitive loss by administering one or more compositions as described herein to the individual in accordance with the administration discussion herein. In an alternative embodiment, cognitive enhancement may also benefit those individuals even useful under non-pathological conditions, e.g., those individuals who do not present with cognitive impairment.

Further to the neuroregenerative activity, it has been found that the compounds according to the present invention act anti-apoptotic on neuronal cells and thereby they can enhance the viability of neurons. Therefore, the compounds referred to in accordance with the present invention can be regarded as neuroprotective compounds. The term "neuroprotective compounds" refers to substances which have a protective effect on neural cells and/or the brain tissue in total either in vitro (cell culture systems) or in vivo (animal models for ischemic, hypoxic and/or neurodegenerative diseases, such as stroke, ALS or Huntington's disease) or in human patients.

Accordingly, the compounds (N-(3',4'-methylenedioxycinnamoyl)-anthranilic acid, 2-(3',4'-dimethoxycinnamoylamino)-toluene, N-cinnamoyl-anthranilic acid, 2-(3',4'-dimethoxycinnamoylamino)-anisole, and 3-(4'-chlorocinnamoylamino)-anisole have been found to be useful for the therapy of neurological conditions, preferably due to their ability to enhance neurogenesis and/or protect neurons from cell death.

The neuroregenerative effect of the compounds according to the present invention can be assayed in vitro by measuring their stimulation of the differentiation of neuronal stem cells. Therefore, adult neuronal stem cells have to be isolated and subsequently cultivated in a suitable culture medium. After several passages in vitro the stem cells have to be co-transfected with a DNA construct coding for two discriminable reporter proteins one of them under the control of a constitutive promoter and the other under the control of a beta-III-tubulin promoter. The beta-III-tubulin promoter is induced during the process of neuronal stem cell differentiation; therefore the reporter protein under the control of this promoter serves as a marker for the stem cell differentiation in such an in vitro dual reporter assay (Schneider et al., J Clin Investigation 2005; 115:2083-2098). The person skilled in the art knows several suitable constitutive promoters such as the SV40-promoter, the TK-promoter, or the CMV-promoter. As discriminable reporter proteins for such a dual reporter assay one can use discriminable luciferases such as firefly luciferase and Renilla luciferase. The transfected cells have to be incubated with the test compound and with positive and negative controls for normalization and background correction. As a positive control compound one can use retinoic acid (Guan et al., Cell Tissue Res. 2001; 305:171-176*;* Jacobs et al., Proc. Natl. Acad. Sci. USA 2006; 103:3902-3907), a suitable negative control is the sham treatment of the cells. The neroregenerative activity of the test compound is determined as the ratio of the signal of the beta-III-tubulin promoter controlled reporter versus the signal of the constitutively expressed reporter and is expressed as fold from the control. This assay is suitable to assess the neuroregenerative activity of tranilast and derivatives thereof, but also can be used to screen for other potential neuroregenerative compounds. Preferably, such a screening can be used to test compounds which are already known to be suitable as a medicament in terms of its toxicology, bioavailability, solubility, stability etc.

A more detailed specification of such an in vitro assay for the assessment of neuroregenerative effect is given in Examples 1 and 2.

Neuroprotection is attainable via the inhibition of apoptosis of affected neurons. The anti-apoptotic effect of tranilast and its derivatives can be assayed in vitro by measuring the caspase-3 and caspase-7 activities. These members of the cysteine aspartic acid-specific protease (caspase) family play key effector roles in apoptosis in mammalian cells. Cortical neurons have to be isolated and subsequently cultivated in a suitable culture medium. The cells have to be incubated with the test compound in the presence of an apoptotic substance such as staurosporine. Parallel approaches with staurosporine alone and sham treatment of the cells serve as negative and positive controls. The assay allows evaluating whether the test compound can compensate either completely or partially for the apoptotic effect of staurosporine. This assay is suitable to assess the neuroprotective activity of tranilast and derivatives thereof, but also can be used to screen for other potential neuroprotective compounds. Preferably, such a screening can be used to test compounds which are already known to be suitable as a medicament in terms of its toxicology, bioavailability, solubility, stability etc.

A more detailed specification of such an in vitro assay for the assessment of an anti-apoptotic effect on neurons is given in Examples 3 and 4.

The viability assay allows for further assessment of test compounds regarding their effect on cells such as neurons. Therefore, a stably transfected cell line (e.g. SH-SY5Y cells) with a reporter construct (e.g. a luciferase construct) has to be incubated with the test compound and in parallel with positive and negative controls (e.g. staurosporine and sham, respectively). Subsequently, the signal generated by the reporter construct serves as a readout for the viability of the cells in the presence of the test compound. The assay enables the evaluation of potential neurotoxic effects of the test compound itself. The inventors found that tranilast and derivatives thereof have no cytotoxic effect in this assay.

A more detailed specification of such an in vitro assay for the assessment of an effect on the viability of neurons is given in Example 5 and 6.

Animal models for certain neurological conditions and diseases can be used for the further evaluation of the in vivo effect of a test compound. The rat MCAO model (middle cerebral occlusion; filament model) is an animal model for stroke, where a broad variety of different neuron types is damaged by ischemia/hypoxia. Further animal models for various neurological conditions are well known to the person skilled in the art. Such animal models are for example but not limited to SOD1G93A-transgenic mouse for amyotrophic lateral sclerosis (Almer et al., J Neurochem 1999; 72:2415-2425), APP-transgenic mice for Alzheimer's disease (Janus & Westaway, Physiol Behav 2001; 73:873-886), MPTP-treated animals for Parkinson's disease (Smeyne & Jackson-Lewis, Brain Res Mol Brain Res. 2005; 134:57-66*),* exon-1-huntingtin-transgenic mice for Huntington's disease (Sathasivam et al., Philos Trans R Soc Lond B Biol Sci. 1999; 354:963-969), and FRDA-transgenic mice for Friedreich ataxia (Al-Mahdawi Genomics 2006:88:580-590). Behavioural scores and/or pathological outcome of the animals treated with the test compound have to be compared with sham treated animals.

The compounds referred to in accordance with the present invention, alone, in combination with each other, and/or in combination with one or more additional compounds can be used for treating and/or preventing neurological conditions where there is a need for neuroregeneration. These neurological conditions preferably comprise cerebral ischemia (such as stroke, traumatic brain injury, or cerebral ischemia due to cardiocirculatory arrest), amyotrophic lateral sclerosis, glaucoma, Alzheimer's disease, Parkinson's disease, neurodegenerative trinucleotide repeat disorders (such as Huntington's disease), neurodegenerative lysosomal storage diseases, multiple sclerosis, spinal cord injury, spinal cord trauma, dementia, schizophrenia, depression, and peripheral neuropathy.

In a preferred embodiment of the uses and methods of the present invention, the compounds referred to in accordance with the present invention, alone, in combination with each other, and/or in combination with one or more additional factors can be used for treating and/or preventing neurological conditions where there is a need for neuroregeneration which are based on ischemia and/or hypoxia of the neural tissue, such as cerebral ischemia (e.g. stroke, traumatic brain injury, or cerebral ischemia due to cardiocirculatory arrest), glaucoma, spinal cord injury, or spinal cord trauma by providing protection and regeneration neuronal cells in those patients with the condition. The common pathological and protective processes active in these neurological conditions indicate that a therapy the compounds according to the present invention will be comparably effective.

In a further embodiment of the uses and methods of the present invention, the compounds referred to in accordance with the present invention, alone, in combination with each other, and/or in combination with one or more additional factors can be used for treating and/or preventing neurological conditions selected of the group consisting of glaucoma, neurodegenerative trinucleotide repeat disorders (e.g. Huntington's disease), neurodegenerative lysosomal storage diseases, spinal cord injury, spinal cord trauma, dementia, schizophrenia, depression, and peripheral neuropathy.

Additionally, the compounds referred to in accordance with the present invention, alone, in combination with each other, and/or in combination with one or more additional factors can be used to enhance learning and memory, e.g. in the case of non-pathological conditions such as age-related memory loss, mild cognitive impairment, or age-related cognitive decline.

By "treating" is meant the slowing, interrupting, arresting or stopping of the progression of the disease or condition and does not necessarily require the complete elimination of all disease symptoms and signs. Moreover, as will be understood by those skilled in the art, such treatment is usually not intended to effective for 100% of the subjects to be treated. The term, however, requires that a statistically significant portion of subjects can be efficiently treated, i.e. the symptoms and clinical signs can be ameliorated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001.

For certain neurological diseases (e.g. amyotrophic lateral sclerosis and Huntington's disease) a predisposition can be determined based on genetic markers or familial accumulation of the disease. For other neurological conditions (e.g. cerebral ischemia such as stroke) various risk factors such as smoking, malnutrition, or certain blood levels are known by the person skilled in art. The treatment of such a neurological disease is most promising when starting very early even before the onset of the clinical symptoms (Ludolph, J Neurol 2000; 247:VI/13-VIl18). At that stage of the disease the molecular and cellular damage already has begun. It is obvious that a neuroregenerative and/or neuroprotective compound is most effectively preventing the neurological condition or disease when given as soon as the cellular damage (i.e. the loss of neurons) just has started.
Compounds to be used in accordance with the present invention, such as tranilast, which are well tolerated even upon long-term application can be used as a prophylaxis to treat those patients that are at risk of developing such a neurological conditions or diseases. Prophylactic administration should be administered in a way to establish a sustained level of compound in an effective amount for the stimulation of neurogenesis and for neuroprotection.

"Preventing" is intended to include the prophylaxis of the neurological disease or condition, wherein "prophylaxis" is understood to be any degree of inhibition of the time of onset or severity of signs or symptoms of the disease or condition, including, but not limited to, the complete prevention of the disease or condition. Moreover, as will be understood by those skilled in the art, such a prevention is usually not intended to effective for 100% of the subjects to be subjected to it. The term, however, requires that a statistically significant portion of subjects can be efficiently prevented from developing a disease or condition referred to herein in the future. Whether a portion is statistically significant can be determined without further ado by the person skilled in the art using various well known statistic evaluation tools as referred to elsewhere in this specification.

The compound referred to in accordance with the present invention and combinations thereof, may be administered in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application.

The route of administration can include the typical routes including, for example, orally, subcutaneously, transdermally, intradermally, rectally, vaginally, intramuscularly, intravenously, intraarterially, by direct injection to the brain, and parenterally. The pharmaceutical preparation also may be adapted for topical use. In addition, in some circumstances, pulmonary administration may be useful, e.g., pulmonary sprays and other respirable forms.

In addition to pulmonary sprays, intranasal (IN) delivery (for example by nasal sprays) is a preferred application mode of delivering the compositions of the present invention for neurological/psychiatric conditions. Intranasal delivery is well suited to serve as application mode of proteins and peptides, and is very convenient to use, especially for long-term treatments. Examples for the use of intranasal delivery (nasal sprays) for applying peptides or proteins to the brain can be found in: (Lyritis & Trovas, Bone 2002; 30:71S-74S*,* Dhillo & Bloom, Curr Opin Pharmacol 2001; 1:651-655*,* Thorne & Frey, Clin Pharmacokinet 2001; 40:907-946*,* Tirucherai et al., Expert Opin Biol Ther 2001; 1:49-66, Jin et al., Ann Neurol 2003 ; 53:405-409*,* Lemere et al., Neurobiol Aging 2002; 23:991-1000*,* Lawrence, Lancet 2002; 359:1674, Liu et al., Neurosci Lett 2001; 308:91-94). For intranasal application, the compounds described herein can be combined with solvents, detergents and substances that increase penetration of the nasal epithelium or delivery into blood vessels, such as drugs that widen nasal blood vessel, increase perfusion, etcetera.

The above-described substances according to the invention can be formulated for medical purposes according to standard procedures available in the art, e.g., a pharmaceutically acceptable carrier (or excipient) can be added. A carrier or excipient can be a solid, semisolid or liquid material which can serve as a vehicle or medium for the active ingredient. The proper form and mode of administration can be selected depending on the particular characteristics of the product selected, the disease, or condition to be treated, the stage of the disease or condition, and other relevant circumstances (Remington's Pharmaceutical Sciences, Mack Publishing Co. (1990)). The proportion and nature of the pharmaceutically acceptable carrier or excipient are determined by the solubility and chemical properties of the substance selected the chosen route of administration, and standard pharmaceutical practice. Topical formulations may also contain penetration enhancers such as oleic acid, propylene glycol, ethanol, urea, lauric diethanolamide or azone, dimethyl sulphoxide, decylmethyl sulphoxide, or pyrrolidone derivatives. Liposomal delivery systems may also be used.

Preferably, the subject to be treated in the uses and methods of the present invention is a mammal, more preferably, a guinea pig, dog, cat, rat, mouse, horse, cow, sheep, monkey or chimpanzee, most preferably it is a human.

A therapeutically effective amount of the compound for treating a neurological condition when the factors are used either singularly or in combination should be used in an amount that results in a neuroprotective and/or neuroregenerative effect. Such an effect can be assessed by the assays described herein. The amount preferably range from about 10 mg to 1000 mg per compound or as a combination and can be determined based on age, race, sex, mode of administration and other factors based on the individual patient. The compound can be administered as a single bolus or repeatedly, e.g. as a daily dose, depending on treated neurological condition and on the route of administration. When the compounds are administered in combination, they may be premixed prior to administration, administered simultaneously, or administered singly in series. In certain embodiments of treating neurological conditions as described herein, higher doses of the compounds described herein can be especially useful, for example, at least 1000 mg daily, at least 2000 mg daily, or at least 5000 mg daily may be used. The person skilled in the art knows approaches to infer a human dosage from animal models (Boxenbaum & DiLea, J Clin Pharmacol 1995; 35:957-966). For the treatment of allergic diseases tranilast is usually applied to human patients via oral administration in an amount of approximately 100 mg to 300 mg daily. At this dosage tranilast is usually well tolerated. Higher doses of tranilast (300 mg to 1000 mg daily) have been reported for the prevention of restenosis after percutaneous transluminal coronary angioplasty (EP 588518).

In the case of chronic neurodegenerative processes, such as ALS and dementia, treatment will more likely consist in one daily or preferably use slow-release formulations.

In another embodiment, the present invention also provides a device especially suited for slow release and constant long-term application that may be an implanted mini-pump, preferably implanted sub-cutaneously (for example, as described in Edith Mathiowitz; (Ed.), Encyclopedia of Controlled Drug Delivery, John Wily & Sons 1999; 2:896-920*).* Such pumps are known to be useful in insulin therapy. Examples of such pumps include those manufactured/distributed by Animas, Dana Diabecare, Deltec Cozm, Disetronic Switzerland, Medtronic, and Nipro Amigo as well as those described, for example, in U.S. Pat. Nos. 5,474,552*;* 6,558,345*;* 6,122,536; 5, 492, 534*; and* 6,551,276.

In one embodiment, the medicament can further comprise one or more additional factors. "Additional factors" according to the invention are any substances that further support the beneficial effect of the medicament. This support can be either cumulative or synergistic. Suitable additional factors are e.g. factors with neuroprotective effects such as erythropoietin, BDNF, VEGF, CTNF, G-CSF and GM-CSF or inflammation modulating factors. Adding bradykinin or analogous substances to an intravenous application of any preparation will support its delivery to the brain, or spinal cord (Emerich et al., Clin Pharmacokinet 2001; 40:105-123*;* Siegal et al., Clin Pharmacokinet 2002; 41:171-186). Antiapoptotic agents or agents that facilitate the passage over the blood brain barrier may also be used. A person skilled in the art is familiar with the additional factors that are beneficial for the treatment of the individual illnesses.

Also preferred are modifications or pharmaceutical formulations of the medicament according to the invention that increase its ability to cross the blood-brain-barrier, or shift its distribution coefficient towards brain tissue.

In the present application it is demonstrated that the above mentioned compounds trigger the differentiation of neuronal stem cells towards a neuronal phenotype of the cells. The importance in neurogenesis reasons the applicability and usefulness of treatment with the compounds according to the present invention in all facets of neurodegenerative diseases, and all conditions where neurons die. In contrast to acting on endogenous stem cells in the brain for the treatment of neurological conditions, tranilast or derivatives thereof can be applied to in vitro manipulations of stem cells, for example differentiation and proliferation.

Therefore in another embodiment of the invention, the compounds can be used to facilitate culturing of stem cell, such as, for example, neural stem cells. In this method, the compounds can be added to the media and premixed before adding to the cells or can be added into the media in which the cells are being cultured.

Thus, the present invention relates, furthermore, to a method for the in vitro differentiation of stem cells excluding human embryonic stem cells comprising contacting stem cells with at least on compound as defined above. Preferably, the stem cells used in the method are neuronal stem cells.

The differentiated stem cells obtainable by the aforementioned method are useful for the preparation of a pharmaceutical composition for treating a neuronal condition as specified elsewhere in this specification.

Accordingly, in one embodiment of the present invention relates to stimulating the growth and differentiation of neuronal stem cells or precondition neuronal stem cells prior to implantation into a mammal using the aforementioned compounds according to the present invention. A further embodiment of this method is to utilize these neuronal stem cells in methods for treating neurological disease as described herein, preferably in methods which provide a neuroprotective effect when the neuronal stem cells are administered to the individual.

The stem cells can be administered intravenously or intraarterially. It has been shown, for example, in cerebral ischemia or traumatic brain injury, that bone marrow stromal cells injected i.v. find their way to target areas in the brain (Mahmood et al., Neurosurgery 2001 ; 49: 1196-1204*;* Lu et al., J Neurotrauma 2001 ; 18;813-819; Lu et al., Cell Transplant 2002 ; 11:275-281*;* Li et al., Neurology 2002; 59:514-523*).* Stem cells may thus be treated by tranilast or derivatives thereof in vitro, and then injected via different routes to patients with any of the diseases described herein.

Finally, the present invention encompasses a kit comprising the compounds referred to in accordance with the present invention in a suitable formulation for the in vitro differentiation of neuronal stem cells and other cells which are derived from neuronal stem cells.

### Brief description of the figures

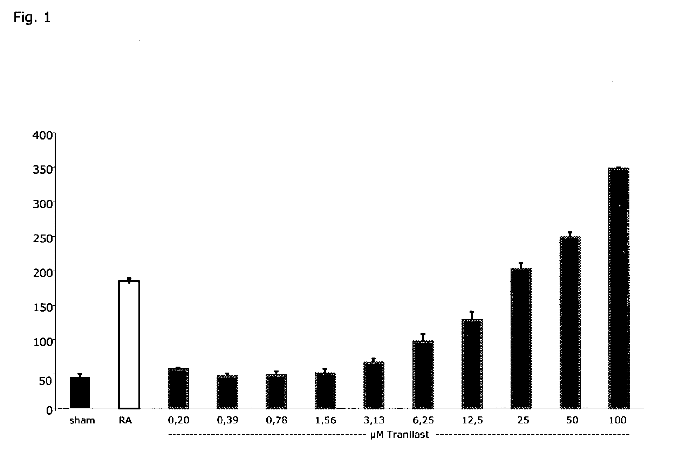

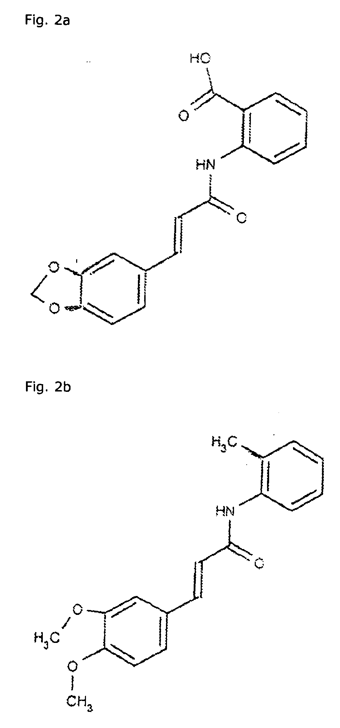

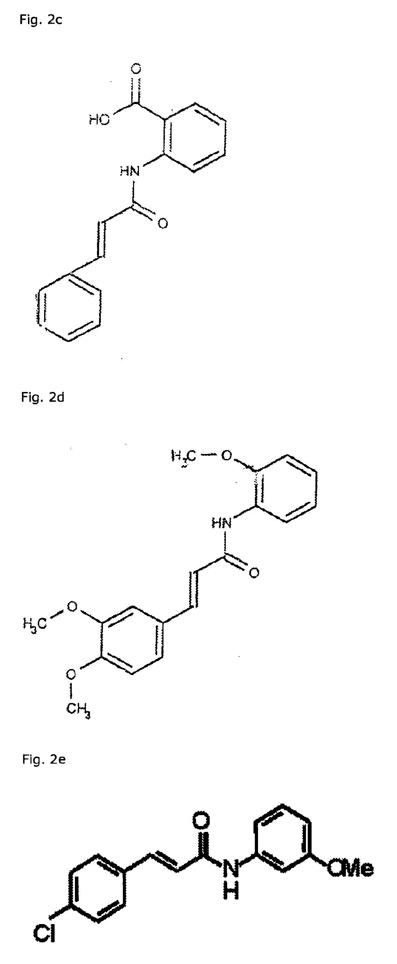

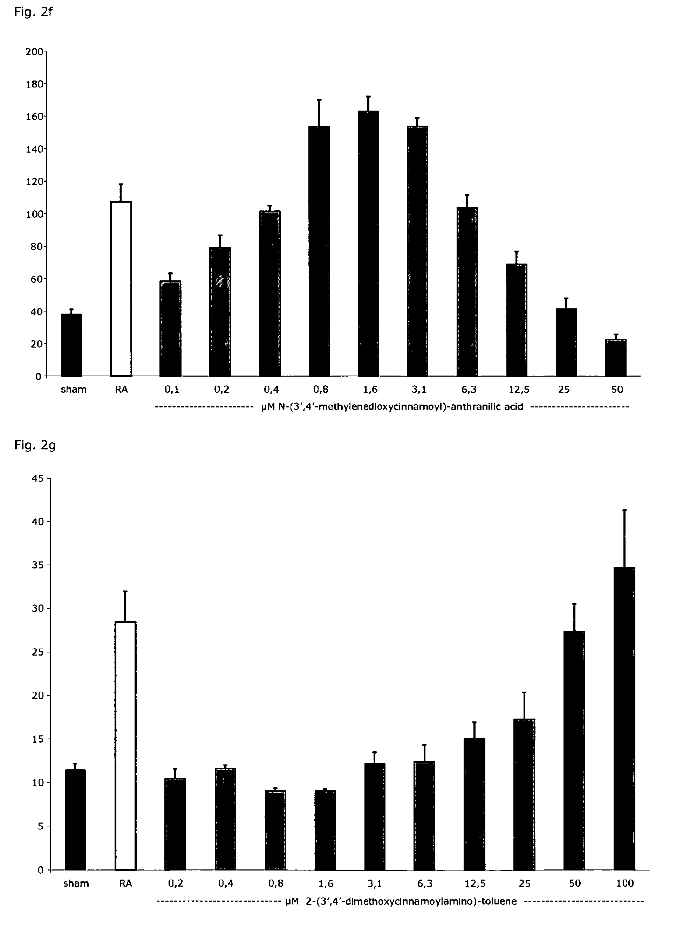

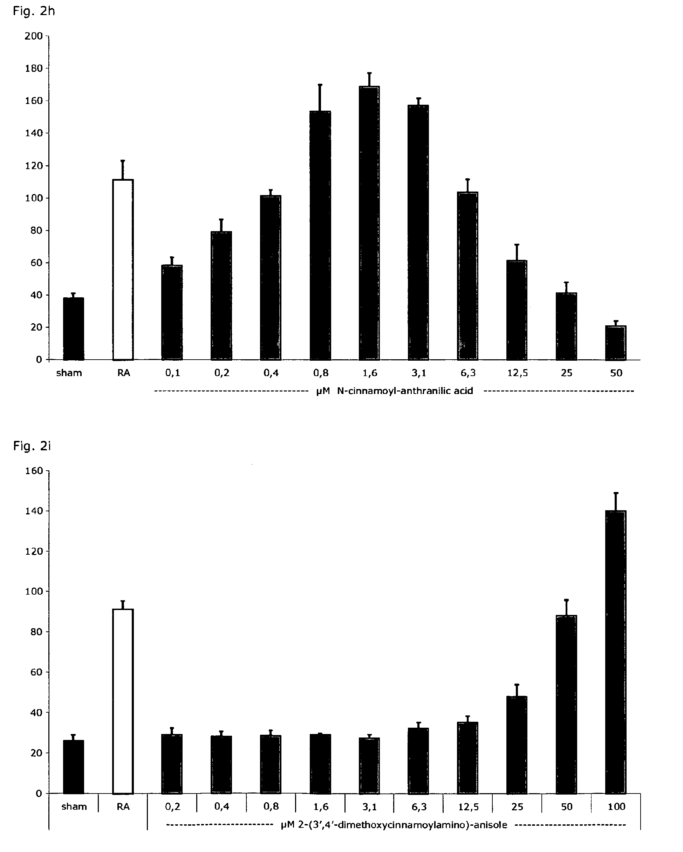

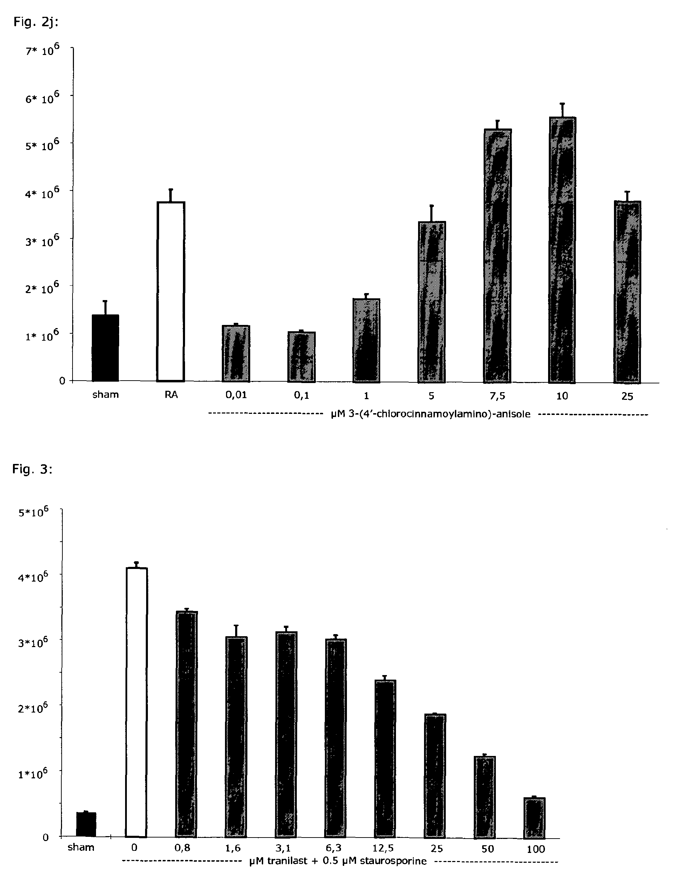

Figure 1:
   Tranilast significantly stimulates the differentiation of neural stem cells to neurons in a dose dependent manner.
   The differentiation of neural stem cells to neurons is measured by the relative induction of the beta-III-tubulin promoter controlled firefly luciferase activity versus the constitutively expressed Renilla luciferase activity. The effect of tranilast in a range from 0,2 µM to 100 µM is shown in comparison to the sham treated cells and to the 1 µM retinoic acid (RA) treated cells. Ratios are given in means with SEM as error bar.
Figure 2 a:
   Chemical formula of N-(3',4'-methylenedioxycinnamoyl)-anthranilic acid
Figure 2 b:
   Chemical formula of 2-(3',4'-dimethoxycinnamoylamino)-toluene
Figure 2 c:
   Chemical formula of N-cinnamoyl-anthranilic acid
Figure 2 d:
   Chemical formula of 2-(3',4'-dimethoxycinnamoylamino)-anisole
Figure 2 e:
   Chemical formula of 3-(4'-chlorocinnamoylamino)-anisole
Figure 2 f:
   N-(3',4'-methylenedioxycinnamoyl)-anthranilic acid significantly stimulates the differentiation of neural stem cells to neurons in a dose dependent manner. Analogously to Fig. 1, the effect of N-(3',4'-methylenedioxycinnamoyl)-anthranilic acid (Fig. 2 a) in a range from 0,1 µM to 50 µM is shown in comparison to the sham treated cells and to the 1 µM retinoic acid (RA) treated cells. Ratios are given in means with SEM as error bar.
Figure 2 g:
   2-(3',4'-dimethoxycinnamoylamino)-toluene significantly stimulates the differentiation of neural stem cells to neurons in a dose dependent manner.
   Analogously to Fig. 1, the effect of 2-(3',4'-dimethoxycinnamoylamino)-toluene (Fig. 2 b) in a range from 0,2 µM to 100 µM is shown in comparison to the sham treated cells and to the 1 µM retinoic acid (RA) treated cells. Ratios are given in means with SEM as error bar.
Figure 2 h:
   N-cinnamoyl-anthranilic acid significantly stimulates the differentiation of neural stem cells to neurons in a dose dependent manner.
   Analogously to Fig. 1, the effect of N-cinnamoyl-anthranilic acid (Fig. 2 c) in a range from 0,1 µM to 50 µM is shown in comparison to the sham treated cells and to the 1 µM retinoic acid (RA) treated cells. Ratios are given in means with SEM as error bar.
Figure 2 i:
   2-(3',4'-dimethoxycinnamoylamino)-anisole significantly stimulates the differentiation of neural stem cells to neurons in a dose dependent manner.
   Analogously to Fig. 1, the effect of 2-(3',4'-dimethoxycinnamoylamino)-anisole (Fig. 2 d) in a range from 0,2 µM to 100 µM is shown in comparison to the sham treated cells and to the 1 µM retinoic acid (RA) treated cells. Ratios are given in means with SEM as error bar.
Figure 2 j:
   3-(4'-chlorocinnamoylamino)-anisole significantly stimulates the differentiation of neural stem cells to neurons in a dose dependent manner.
   Analogously to Fig. 1, the effect of 3-(4'-chlorocinnamoylamino)-anisole (Fig. 2 e) in a range from 0,01 µM to 25 µM is shown in comparison to the sham treated cells and to the 1 µM retinoic acid (RA) treated cells. Ratios are given in means with SEM as error bar.
Figure 3:
   Tranilast has a significant anti-apoptotic effect on neurons in a dose dependent manner.
   After incubation of SH-SY5Y neuronal cells with either staurosporine (0,5 µM) alone or in combination with tranilast (0,8 µM to 100 µM) the subsequent apoptosis of the cells is measured using the Caspase-Glo 3/7 Assay Kit (Promega). Arbitrary luminometric signals of the assay as a measure for the apoptosis are given in means with SEM as error bar. As negative control the luminometric signal of sham treated cells is shown.
Figure 4a:
   Derivatives of tranilast have a significant anti-apoptotic effect on neurons.
   After incubation of SH-SY5Y neuronal cells with tranilast (tnl) and derivatives thereof (N-(3',4'-methylenedioxycinnamoyl)-anthranilic acid (A; Fig. 2a), 2-(3',4'-dimethoxycinnamoylamino)-toluene (B; Fig. 2b), N-cinnamoyl-anthranilic acid (C; Fig. 2c), and 2-(3',4'-dimethoxycinnamoylamino)-anisole (D; Fig. 2d) each in a concentration of 1 µM each alone and in co-incubation with staurosporine (SP; 0,5 µM) the subsequent apoptosis of the cells is measured using the Caspase-Glo 3/7 Assay Kit (Promega). The degree of apoptosis is compared with the one of sham treated cells (negative control) and of staurosporine (SP; 0,5 µM) treated cells (positive control). Arbitrary luminometric signals of the assay as a measure for the apoptosis are given in means with SEM as error bar. Tranilast and derivatives thereof exhibit a significant reduction of the staurosporine induced apoptosis.
Figure 4b:
   Derivatives of tranilast have a significant anti-apoptotic effect on neurons.
   After incubation of SH-SY5Y neuronal cells with 3-(4'-chlorocinnamoylamino)-anisole (E; Fig. 2e)) in a concentration of 1 µM alone and in co-incubation with staurosporine (SP; 0,1 µM) the subsequent apoptosis of the cells is measured using the Caspase-Glo 3/7 Assay Kit (Promega). The degree of apoptosis is compared with the one of sham treated cells (negative control) and of staurosporine (SP; 0,1 µM) treated cells (positive control). Arbitrary luminometric signals of the assay as a measure for the apoptosis are given in means with SEM as error bar. 3-(4'-chlorocinnamoylamino)-anisole exhibit a significant reduction of the staurosporine induced apoptosis.
Figure 5:
   Tranilast does not diminish the viability of neuronal cells.
   The viability of SH-SY5Y neuronal cells after incubation with tranilast (final concentration of 0,8 µM to 50 µM) has been determined. Therefore, the cells have been stably transfected with a constitutively expressing luciferase construct prior to the incubation. After the incubation the viability of the cells has been evaluated by measuring the luciferase activity. Arbitrary luminometric signals of the assay as a measure for the remaining viability are given in means with SEM as error bar. As negative and positive control the luminometric signal of sham and staurosporine (SP; 0,5 µM) treated cells is shown, respectively.
Figure 6:
   Derivatives of tranilast do not diminish the viability of neuronal cells.
   The viability of SH-SY5Y neuronal cells after incubation with tranilast (tnl) and derivatives thereof (N-(3',4'-methylenedioxycinnamoyl)-anthranilic acid (A; Fig. 2a), 2-(3',4'-dimethoxycinnamoylamino)-toluene (B; Fig. 2b), N-cinnamoyl-anthranilic acid (C; Fig. 2c), and 2-(3',4'-dimethoxycinnamoylamino)-anisole (D; Fig. 2d)) each in a concentration of 1 µM and of 10 µM has been determined. Therefore, the cells have been stably transfected with a constitutively expressing luciferase construct prior to the incubation. After the incubation the viability of the cells has been evaluated by measuring the luciferase activity. Arbitrary luminometric signals of the assay as a measure for the remaining viability are given in means with SEM as error bar. As negative and positive control the luminometric signal of sham and staurosporine (SP; 0,5 µM) treated cells is shown, respectively.

### Examples

The following Examples are provided herein for purposes of illustration only and are not intended to be limiting unless otherwise specified.

### Example 1:

In vitro differentiation assay on adult neuronal stem cells with tranilast

Neural stem cells were isolated from the subventricular zone of 4 week-old male Wistar rats as described (Maurer et al, Proteome Sci. 2003; 1:4). Cells were cultivated in Neurobasal medium (Invitrogen) supplemented with B27 (Invitrogen), 2mM L-glutamine, 100units/ml penicillin, 100units/ml streptomycin, 20ng/ml endothelial growth factor (EGF), 20ng/ml fibroblast growth factor-2 (FGF-2), and 2µg/ml heparin. Once a week the neural stem cells were passaged and experiments were performed after 4 weeks in vitro. For DNA transfection, the cells were dissociated and plated on poly-L-ornithin/laminin-coated 96-well plates at a density of 50.000 cells/well. Cotransfection with the pGL3-p-ßIII-tubulin vector (100ng/well) and the pRL SV40 vector (100ng/well) was carried out according to the FuGene6 Transfection protocol (Roche). The pRL SV40 vector (Promega) served as an internal control vector.

To amplify the class III ß-tubulin gene promoter (fragment -450 - +54), rat genomic DNA was used as a template for PCR (Dennis et al., Gene 2002;294:269-277). The amplified fragment was inserted into the Mlu I/Xho I site of the pGL3-Basic firefly luciferase reporter vector (Promega) to generate the pGL3-p-ßIII-tubulin experimental vector.

After overnight incubation, the plate was decanted and given in 8-fold replicates fresh medium containing tranilast (Sigma) or vehicle. As a positive control for in vitro differentiation, stem cells were treated by adding 1 µM retinoic acid (Sigma) to the medium missing the mitogens.

After 48hrs, the cells were harvested to prepare the cellular extracts for luciferase assay following the directions of the manufacturer (Promega). As the cells were cotransfected with the firefly and the Renilla luciferase, the Dual-Luciferase Reporter Assay System (Promega) was used and the ratio of luminescence signals from the reaction mediated by firefly luciferase to those from the reaction mediated by Renilla luciferase were measured with a luminometer (Berthold Technologies, Mithras LB 940). Variations in the transfection efficiency were normalized using the luminescence signals from the constitutively expressed Renilla luciferase.

Tranilast was used in a range from 0,2 to 100 µM final concentration. The relative induction of the class III ß-tubulin gene promoter controlled luciferase signal as a measurement for the neural stem cell differentiation is shown in Fig. 1. The results indicate that tranilast significantly stimulates the differentiation of neural stem cells to neurons and therefore acts neuroregenerative.

### Example 2:

### In vitro differentiation assay on adult neuronal stem cells with derivatives of tranilast

Analogously to Example 1, various derivatives of tranilast (N-(3',4'-methylenedioxycinnamoyl)-anthranilic acid (Fig. 2a (ChemBridge Corporation)), 2-(3',4'-dimethoxycinnamoylamino)-toluene (Fig. 2b (ChemBridge Corporation)), N-cinnamoyl-anthranilic acid (Fig. 2c (ChemBridge Corporation)), 2-(3',4'-dimethoxycinnamoylamino)-anisole (Fig. 2d (Ambinter SARL)), 3-(4'-chlorocinnamoylamino)-anisole (Fig. 2e (Sigma))) have been analysed for their activity to stimulate the differentiation of neural stem cells to neurons. The derivatives were used in a range from 0,1 to 100 µM final concentration. The relative induction of the class III ß-tubulin gene promoter controlled luciferase signal as a measurement for the neural stem cell differentiation is shown for these derivatives of tranilast in Fig. 2f-j, respectively. The results indicate that all tested derivatives of tranilast significantly stimulate the differentiation of neural stem cells to neurons and therefore act neuroregenerative.

### Example 3:

### In vitro anti-apoptosis assay with tranilast

The neuroprotective effect of compounds can be assayed in vitro by measuring caspase-3 and caspase-7 activities. These members of the cysteine aspartic acid-specific protease (caspase) family play a key effector role in apoptosis in mammalian cells.

The human neuroblastoma cell-line SH-SY5Y (American Type Culture Collection) maintained in DMEM with high glucose supplemented with 20% FBS was used for the apoptosis assay. Alternatively one can analyse the apoptosis of primary cortical neurons which can be isolated from E18 rats and subsequently cultivated in suitable culture medium. 2,5 x 10⁴ SH-SY5Y cells were seeded in 96-well plates and stimulated 24 h later with tranilast or vehicle in the presence of the apoptosis inducing compound staurosporine (0,5 µM, Calbiochem). After 5h incubation at 37 °C, caspase-Glo 3/7 reagent (Promega) containing luciferase and luminogenic substrate was added to the cells. The cleavage of the substrate by caspase-3 and caspase-7 was detected 30 min later using a luminometer (Berthold Technologies, Mithras LB 940). The Luminescence which is proportional to the amount of caspase activity serves as a measure for the apoptosis of the treated neurons. Tranilast was tested in a range from 0,8 µM to 100 µM final concentration. The tranilast treated cells showed a significant reduction of the staurosporine induced apoptosis as shown in Fig. 3 (vehicle (sham): 9 %; staurosporine: 100 %; 0,8 µM tranilast plus 0,5 µM staurosporine: 84 %; 1,6 µM tranilast plus 0,5 µM staurosporine: 74 %; 3,1 µM tranilast plus 0,5 µM staurosporine: 76 %; 6,3 µM tranilast plus 0,5 µM staurosporine: 74 %; 12,5 µM tranilast plus 0,5 µM staurosporine: 58 %; 25 µM tranilast plus 0,5 µM staurosporine: 46 %; 50 µM tranilast plus 0,5 µM staurosporine: 30 %; 100 µM tranilast plus 0,5 µM staurosporine: 15 %). The results indicate that tranilast exhibits a significant anti-apoptotic effect on neurons and therefore acts neuroprotective.

### Example 4:

### In vitro anti-apoptosis assay with derivatives of tranilast.

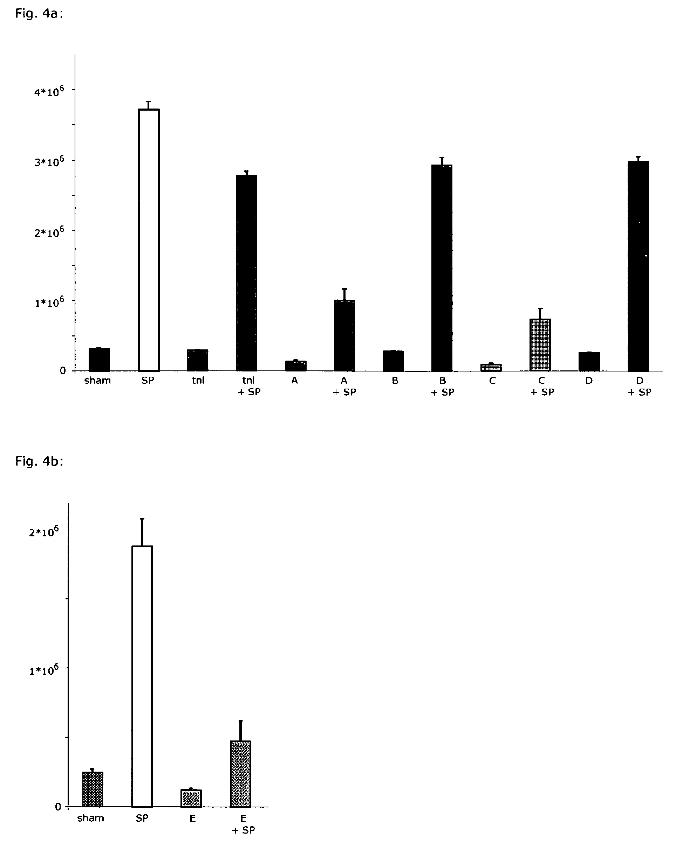

Analogously to Example 3, various derivatives of tranilast (N-(3',4'-methylenedioxycinnamoyl)-anthranilic acid (Fig. 2a (ChemBridge Corporation)), 2-(3',4'-dimethoxycinnamoylamino)-toluene (Fig. 2b (ChemBridge Corporation)), N-cinnamoyl-anthranilic acid (Fig. 2c (ChemBridge Corporation)), and 2-(3',4'-dimethoxycinnamoylamino)-anisole (Fig. 2d (Ambinter SARL))) have been analysed for their anti-apoptotic activity. The SH-SY5Y cells have been incubated with these derivatives (1 µM) either alone or in the presence of staurosporine (0,5 µM). Incubation with staurosporine (0,5 µM) alone served as positive control and incubation with vehicle served as negative control. In the case of 3-(4'-chlorocinnamoylamino)-anisole (Fig. 2e (Sigma)) the SH-SY5Y cells have been incubated with this derivative (1 µM) either alone or in the presence of staurosporine (1 µM). Accordingly, incubation with staurosporine (0,1 µM) alone served as positive control and incubation with vehicle served as negative control. The degree of apoptosis relative to the staurosporine control is shown in Fig. 4 a (vehicle (sham): 9 %; staurosporine (SP): 100 %; tranilast: 8 %; tranilast plus staurosporine: 75 %; derivative A: 3 %; derivative A plus staurosporine: 27 %; derivative B: 7 %; derivative B plus staurosporine: 79 %; derivative C: 2 %; derivative C plus staurosporine: 20 %; derivative D: 7 %; derivative D plus staurosporine: 80 %) and Fig. 4 b (vehicle (sham): 13 %; staurosporine: 100 %; derivative E: 6 %; derivative E plus staurosporine: 25 %). The cells treated with tranilast or derivatives thereof showed a significant reduction of the staurosporine induced apoptosis. The results indicate that tranilast and derivatives thereof exhibit a significant anti-apoptotic effect on neurons and therefore act neuroprotective. Furthermore, neither tranilast nor derivatives thereof exhibited an apoptotic effect by themselves.

### Example 5:

### In vitro viability assay with tranilast

The effect of a compound on the viability of neuronal cells can be assayed in vitro by measuring the survival of neuronal cells transfected with a reporter construct under a constitutive promoter.

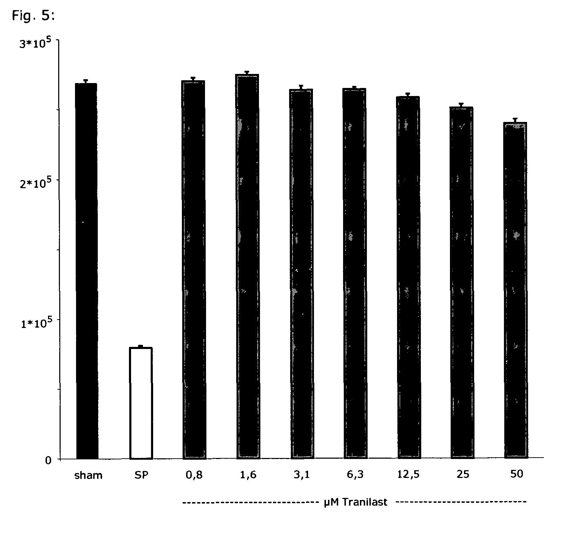

SH-SY5Y neuronal cells stably transfected with a Renilla luciferase construct under the SV40 promoter were seeded in 96-well plates at a density of 2,5 x 10⁴ cells per well. Following overnight incubation the cells were stimulated with medium containing tranilast or vehicle. Staurosporine (0,5 µM) served as positive control. The Renilla luciferase activity was measured 5 h later using a luminometer (Berthold Technologies, Mithras LB 940). Tranilast was tested in a range from 0,8 µM to 50 µM final concentration. The tranilast treated cells did not show any significant effect on the viability of the neuronal cells (Fig. 5). The results indicate that tranilast does not reduce the viability of neuronal cells.

### Example 6:

### In vitro viability assay with derivatives of tranilast

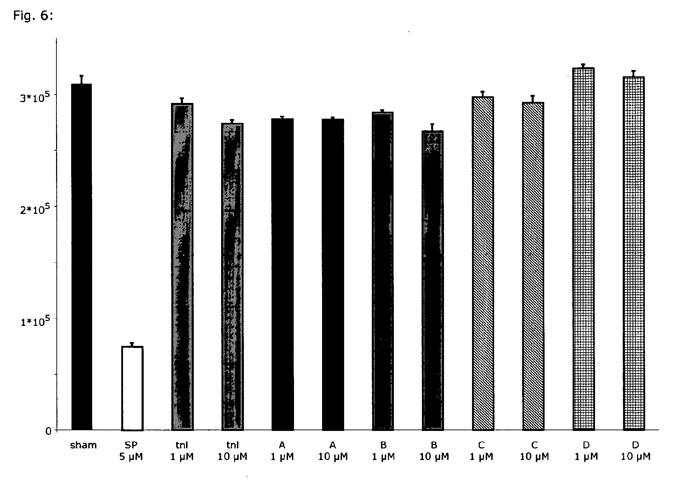

Analogously to Example 5, various derivatives of tranilast (N-(3',4'-methylenedioxycinnamoyl)-anthranilic acid (Fig. 2a (ChemBridge Corporation)), 2-(3',4'-dimethoxycinnamoylamino)-toluene (Fig. 2b (ChemBridge Corporation)), N-cinnamoyl-anthranilic acid (Fig. 2c (ChemBridge Corporation)), and 2-(3',4'-dimethoxycinnamoylamino)-anisole (Fig. 2d (Ambinter SARL))) have been analysed for their effect on the viability of neuronal cells. The derivatives were tested in a final concentration of 1 µM and of 10 µM. The neuronal cells treated with the derivatives of tranilast did not show any significantly reduced viability (Fig. 6). The results indicate that derivatives of tranilast do not reduce the viability of neuronal cells.

## Claims

1. A compound having the following general formula (I) 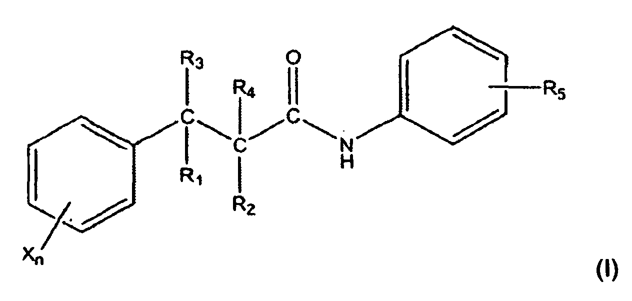 wherein
R₁ and R₂ are independently a hydrogen atom or an alkyl group having from 1 to 6 carbon atoms;
R₃ and R₄ are each hydrogen atoms, or R₃ and R₄ together form a second carbon-to-carbon bond resulting in a cis- or trans-alkene moiety;
R₅ is a group C(O)OR₅ₐ, an alkyl group having from 1 to 6 carbon atoms, or an alkoxy group having from 1 to 6 carbon atoms, whereby R₅ₐ is hydrogen or an alkyl group having from 1 to 6 carbon atoms;
n = 0, 1, 2, or 3; and
each X is independently a hydroxyl group, a halogen atom, a nitro group, an alkyl group having from 1 to 6 carbon atoms, or an alkoxy group having from 1 to 6 carbon atoms;
optionally, two groups X are joined to form an alkylene group having from 1 to 6 carbon atoms, wherein the alkylene group is optionally interrupted by one or more oxygen atoms
for use in a method for treating and/or preventing amyotrophic lateral sclerosis, glaucoma, Alzheimer's disease, Parkinson's disease, neurodegenerative trinucleotide repeat disorders, neurodegenerative lysosomal storage diseases, spinal cord injury, spinal cord trauma, dementia, schizophrenia, or peripheral neuropathy by enhancing or inducing neurogenesis.

2. The compound of claim 1, wherein the compound of the present invention has the general formula (II) 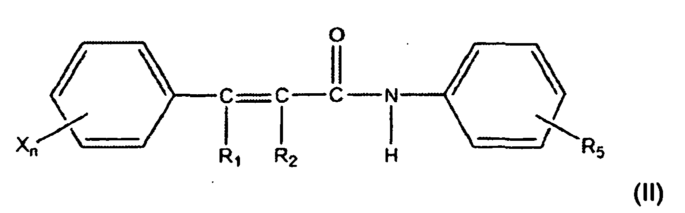 in cis- or trans-form, preferably in trans form, wherein X, n, R₁, R₂, and R₅ have the meaning as indicated in claim 1.

3. The compound of claim 1 or 2, wherein R₁ and R₂ represent hydrogen atoms and any alkoxy or alkyl groups indicated in claim 1 or 2 have from 1 to 4 carbon atoms.

4. The compound of any one of claims 1 to 3, wherein the compound is selected from the group consisting of tranilast, (N-(3',4'-methylenedioxycinnamoyl)-anthranilic acid, 2-(3',4'-dimethoxycinnamoylamino)-toluene, N-cinnamoyl-anthranilic acid, 2-(3',4'-dimethoxycinnamoylamino)-anisole, and 3-(4'-chlorocinnamoylamino)-anisole.

5. A compound as defined in any one of claims 1 to 4 for use in a method for enhancing learning and memory.

6. A method for the in vitro differentiation of stem cells, excluding human embryonic stem cells, comprising contacting stem cells with at least one compound as defined in any one of claims 1 to 4.

7. The method of claim 6, wherein the stem cells are neuronal stem cells.

8. Differentiated stem cells obtainable by the method of claim 6 or 7 for use in a method for treating a neuronal condition.

9. Kit for the in vitro differentiation of neuronal stem cells, excluding human embryonic stem cells, comprising at least one compound as defined in any one of claims 1 to 4.

10. Use of a compound for the preparation of a pharmaceutical composition for treating and/or preventing amyotrophic lateral sclerosis, glaucoma, Alzheimer's disease, Parkinson's disease, neurodegenerative trinucleotide repeat disorders, neurodegenerative lysosomal storage diseases, spinal cord injury, spinal cord trauma, dementia, schizophrenia, or peripheral neuropathy by enhancing or inducing neurogenesis, the compound having the following general formula (I) 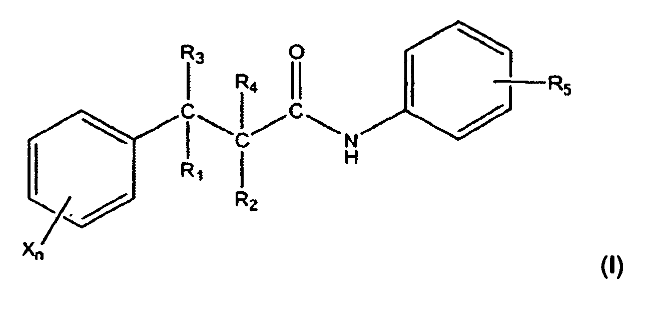 wherein
R₁ and R₂ are independently a hydrogen atom or an alkyl group having from 1 to 6 carbon atoms;
R₃ and R₄ are each hydrogen atoms, or R₃ and R₄ together form a second carbon-to-carbon bond resulting in a cis- or trans-alkene moiety;
R₅ is a group C(O)OR₅ₐ, an alkyl group having from 1 to 6 carbon atoms, or an alkoxy group having from 1 to 6 carbon atoms,
whereby R₅ₐ is hydrogen or an alkyl group having from 1 to 6 carbon atoms;
n=0, 1, 2, or 3; and
each X is independently a hydroxyl group, a halogen atom, a nitro group, an alkyl group having from 1 to 6 carbon atoms, or an alkoxy group having from 1 to 6 carbon atoms;
optionally, two groups X are joined to form an alkylene group having from 1 to 6 carbon atoms, wherein the alkylene group is optionally interrupted by one or more oxygen atoms.

11. The use of claim 10, wherein the compound is defined as in any one of claims 2 to 4.

12. Use of a compound as defined in any one of claims 1 to 4 for the preparation of a pharmaceutical composition for enhancing learning and memory.

13. Use of differentiated stem cells obtainable by the method of claim 6 or 7 for the preparation of a pharmaceutical composition for treating a neuronal condition.

## Patentansprüche

1. Verbindung, die die folgende allgemeine Formel (I) aufweist 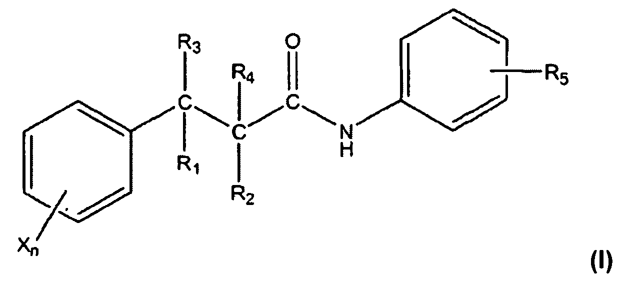 wobei
R₁ und R₂ unabhängig ein Wasserstoffatom oder eine Alkylgruppe, die von 1 bis 6 Kohlenstoffatome aufweist, sind;
R₃ und R₄ jeweils Wasserstoffatome sind, oder R₃ und R₄ zusammen eine zweite Kohlenstoff-zu-Kohlenstoff-Bindung ausbilden, die in einer cis- oder trans-Alkengruppierung resultiert;
R₅ eine Gruppe C(O)OR₅ₐ, eine Alkylgruppe, die von 1 bis 6 Kohlenstoffatome aufweist, oder eine Alkoxygruppe, die von 1 bis 6 Kohlenstoffatome aufweist, ist,
wobei R₅ₐ Wasserstoff oder eine Alkylgruppe, die von 1 bis 6 Kohlenstoffatome aufweist, ist;
n=0, 1, 2 oder 3; und
jedes X unabhängig eine Hydroxylgruppe, ein Halogenatom, eine Nitrogruppe, eine Alkylgruppe, die von 1 bis 6 Kohlenstoffatome aufweist, oder eine Alkoxygruppe, die von 1 bis 6 Kohlenstoffatome aufweist, ist;
optional zwei Gruppen X verbunden sind, um eine Alkylengruppe auszubilden, die von 1 bis 6 Kohlenstoffatome aufweist, wobei die Alkylengruppe optional durch ein oder mehrere Sauerstoffatome unterbrochen ist,
zur Verwendung in einem Verfahren zum Behandeln und/ oder Verhindern von amyotropher Lateralsklerose, Glaukom, Alzheimer-Krankheit, Parkinson-Krankheit, neurodegenerativen Trinukleotid-Repeat-Störungen, neurodegenerativen lysosomalen Speicherkrankheiten, Rückenmarksverletzung, Rückenmarkstrauma, Demenz, Schizophrenie oder peripherer Neuropathie durch Verstärken oder Induzieren von Neurogenese.

2. Verbindung nach Anspruch 1, wobei die Verbindung der vorliegenden Erfindung die allgemeine Formel (II) 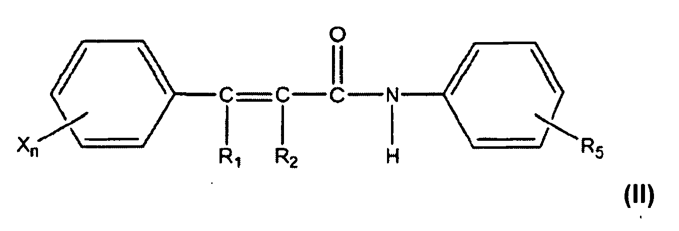 in cis- oder trans-Form, vorzugsweise in trans-Form, aufweist, wobei X, n, R₁, R₂ und R₅ die wie in Anspruch 1 angezeigte Bedeutung haben.

3. Verbindung nach Anspruch 1 oder 2, wobei R₁ und R₂ Wasserstoffatome darstellen und beliebige Alkoxy- oder Alkylgruppen, die in Anspruch 1 oder 2 angegeben sind, von 1 bis 4 Kohlenstoffatome aufweisen.

4. Verbindung nach einem beliebigen der Ansprüche 1 bis 3, wobei die Verbindung ausgewählt ist aus der Gruppe, bestehend aus Tranilast, (N-(3',4'-Methylendioxycinnamoyl)anthranilsäure, 2-(3',4'-Dimethoxycinnamoylamino)toluol, N-Cinnamoylanthranilsäure, 2-(3',4'-Dimethoxycinnamoylamino)anisol und 3-(4'-Chlorcinnamoylamino)anisol.

5. Verbindung, wie in einem beliebigen der Ansprüche 1 bis 4 definiert, zur Verwendung in einem Verfahren zum Steigern von Lernen und Gedächtnis.

6. Verfahren für die In-vitro-Differenzierung von Stammzellen, ausschließlich humaner embryonaler Stammzellen, umfassend In-Kontakt-Bringen von Stammzellen mit mindestens einer Verbindung, wie in einem beliebigen der Ansprüche 1 bis 4 definiert.

7. Verfahren nach Anspruch 6, wobei die Stammzellen neuronale Stammzellen sind.

8. Differenzierte Stammzellen, erhältlich durch das Verfahren nach Anspruch 6 oder 7, zur Verwendung in einem Verfahren zum Behandeln eines neuronalen Zustandes.

9. Kit für die In-vitro-Differenzierung von neuronalen Stammzellen, ausschließlich humaner embryonaler Stammzellen, umfassend mindestens eine Verbindung, wie in einem beliebigen der Ansprüche 1 bis 4 definiert.

10. Verwendung einer Verbindung zur Herstellung einer pharmazeutischen Zusammensetzung zum Behandeln und/ oder Verhindern von amyotropher Lateralsklerose, Glaukom, Alzheimer-Krankheit, Parkinson-Krankheit, neurodegenerativen Trinukleotid-Repeat-Störungen, neurodegenerativen lysosomalen Speicherkrankheiten, Rückenmarksverletzung, Rückenmarkstrauma, Demenz, Schizophrenie oder peripherer Neuropathie durch Verstärken oder Induzieren von Neurogenese, wobei die Verbindung die folgende allgemeine Formel (I) aufweist 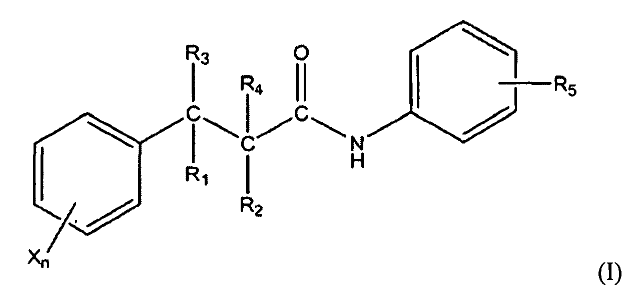 wobei
R₁ und R₂ unabhängig ein Wasserstoffatom oder eine Alkylgruppe, die von 1 bis 6 Kohlenstoffatome aufweist, sind;
R₃ und R₄ jeweils Wasserstoffatome sind, oder R₃ und R₄ zusammen eine zweite Kohlenstoff-zu-Kohlenstoff-Bindung ausbilden, die in einer cis- oder trans-Alkengruppierung resultiert;
R₅ eine Gruppe C(O)OR₅ₐ, eine Alkylgruppe, die von 1 bis 6 Kohlenstoffatome aufweist, oder eine Alkoxygruppe, die von 1 bis 6 Kohlenstoffatome aufweist, ist,
wobei R₅ₐ Wasserstoff oder eine Alkylgruppe, die von 1 bis 6 Kohlenstoffatome aufweist, ist;
n=0, 1, 2 oder 3; und
jedes X unabhängig eine Hydroxylgruppe, ein Halogenatom, eine Nitrogruppe, eine Alkylgruppe, die von 1 bis 6 Kohlenstoffatome aufweist, oder eine Alkoxygruppe, die von 1 bis 6 Kohlenstoffatome aufweist, ist;
optional zwei Gruppen X verbunden sind, um eine Alkylengruppe auszubilden, die von 1 bis 6 Kohlenstoffatome aufweist, wobei die Alkylengruppe optional durch ein oder mehrere Sauerstoffatome unterbrochen ist.

11. Verwendung nach Anspruch 10, wobei die Verbindung wie in einem beliebigen der Ansprüche 2 bis 4 definiert ist.

12. Verwendung einer Verbindung wie in einem beliebigen der Ansprüche 1 bis 4 definiert zur Herstellung einer pharmazeutischen Zusammensetzung zum Steigern von Lernen und Gedächtnis.

13. Verwendung von differenzierten Stammzellen, erhältlich durch das Verfahren nach Anspruch 6 oder 7, zur Herstellung einer pharmazeutischen Zusammensetzung zum Behandeln eines neuronalen Zustandes.

## Revendications

1. Composé de formule générale (I) 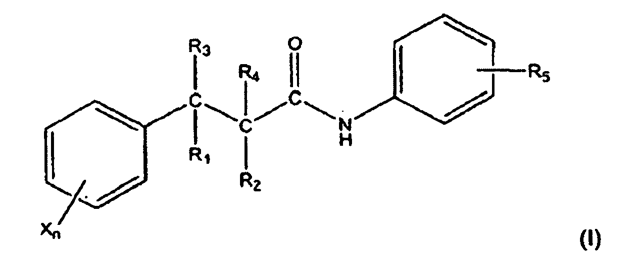 dans laquelle
R₁ et R₂ sont indépendamment un atome d'hydrogène ou un groupe alkyle ayant de 1 à 6 atomes de carbone ;
R₃ et R₄ sont chacun des atomes d'hydrogène, ou R₃ et R₄ ensemble forment une seconde liaison carbone-carbone résultant en un groupement alcène cis ou trans ;
R₅ est un groupe C(O)OR₅ₐ, un groupe alkyle ayant de 1 à 6 atomes de carbone, ou un groupe alcoxy ayant de 1 à 6 atomes de carbone, où R₅ₐ est un atome d'hydrogène ou un groupe alkyle ayant de 1 à 6 atomes de carbone ;
n = 0, 1, 2 ou 3 ; et
chaque X est indépendamment un groupe hydroxyle, un atome d'halogène, un groupe nitro, un groupe alkyle ayant de 1 à 6 atomes de carbone, ou un groupe alcoxy ayant de 1 à 6 atomes de carbone ;
optionnellement, deux groupes X sont liés pour former un groupe alkylène ayant de 1 à 6 atomes de carbone, dans lequel le groupe alkylène est optionnellement interrompu par un ou plusieurs atomes d'oxygène
pour son utilisation dans une méthode de traitement et/ou de prévention de la sclérose latérale amyotrophique, d'un glaucome, de la maladie d'Alzheimer, de la maladie de Parkinson, de maladies neurodégénératives à expansion de triplets, de maladies neurodégénératives de surcharge lysosomale, d'une lésion de la moelle épinière, d'un traumatisme de la moelle épinière, de la démence, de la schizophrénie, ou d'une neuropathie périphérique par amélioration ou induction de la neurogenèse.

2. Composé selon la revendication 1, dans lequel le composé de la présente invention a la formule générale (II) 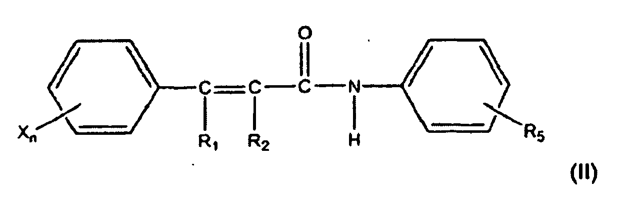 sous la forme cis ou trans, de préférence sous la forme trans, dans laquelle X, n, R₁, R₂ et R₅ ont la signification indiquée dans la revendication 1.

3. Composé selon la revendication 1 ou 2, dans lequel R₁ et R₂ représentent des atomes d'hydrogène et dans lequel tous les groupes alcoxy ou alkyle indiqués dans la revendication 1 ou 2 ont de 1 à 4 atomes de carbone.

4. Composé selon l'une quelconque des revendications 1 à 3, dans lequel le composé est choisi dans le groupe constitué par le tranilast, l'acide (N-(3',4'-méthylènedioxycinnamoyl)-anthranilique, le 2-(3',4'-diméthoxycinnamoylamino)-toluène, l'acide N-cinnamoyl-anthranilique, le 2-(3',4'-diméthoxycinnamoylamino)-anisole, et le 3-(4'-chlorocinnamoylamino)-anisole.

5. Composé selon l'une quelconque des revendications 1 à 4, destiné à être utilisé dans une méthode d'amélioration de l'apprentissage et de la mémoire.

6. Procédé de différenciation in vitro de cellules souches, à l'exclusion de cellules souches embryonnaires humaines, qui comprend la mise en contact de cellules souches avec au moins un composé tel que défini dans l'une quelconque des revendications 1 à 4.

7. Procédé selon la revendication 6, dans lequel les cellules souches sont des cellules souches neuronales.

8. Cellules souches différenciées pouvant être obtenues par le procédé selon la revendication 6 ou 7 et destinées à être utilisées dans une méthode de traitement d'un trouble neuronal.

9. Kit de différenciation in vitro de cellules souches neuronales, à l'exclusion de cellules souches embryonnaires humaines, qui comprend au moins un composé selon l'une quelconque des revendications 1 à 4.

10. Utilisation d'un composé pour la préparation d'une composition pharmaceutique destinée au traitement et/ou à la prévention de la sclérose latérale amyotrophique, d'un glaucome, de la maladie d'Alzheimer, de la maladie de Parkinson, de maladies neurodégénératives à expansion de triplets, de maladies neurodégénératives de surcharge lysosomale, d'une lésion de la moelle épinière, d'un traumatisme de la moelle épinière, de la démence, de la schizophrénie, ou d'une neuropathie périphérique par amélioration ou induction de la neurogenèse, le composé ayant la formule générale (I) suivante 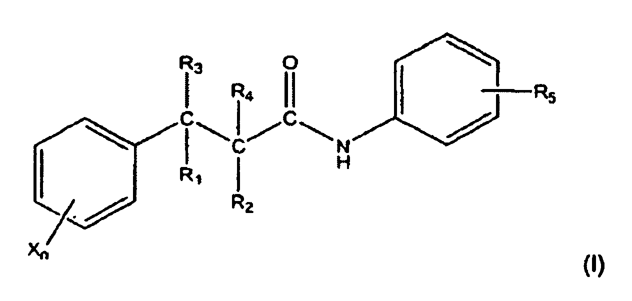 dans laquelle
R₁ et R₂ sont indépendamment un atome d'hydrogène ou un groupe alkyle ayant de 1 à 6 atomes de carbone ;
R₃ et R₄ sont chacun des atomes d'hydrogène, ou R₃ et R₄ ensemble forment une seconde liaison carbone-carbone résultant en un groupement alcène cis ou trans ;
R₅ est un groupe C(O)OR₅ₐ, un groupe alkyle ayant de 1 à 6 atomes de carbone, ou un groupe alcoxy ayant de 1 à 6 atomes de carbone, où R₅ₐ est un atome d'hydrogène ou un groupe alkyle ayant de 1 à 6 atomes de carbone ;
n = 0, 1, 2 ou 3 ; et
chaque X est indépendamment un groupe hydroxyle, un atome d'halogène, un groupe nitro, un groupe alkyle ayant de 1 à 6 atomes de carbone, ou un groupe alcoxy ayant de 1 à 6 atomes de carbone ;
optionnellement, deux groupes X sont liés pour former un groupe alkylène ayant de 1 à 6 atomes de carbone, dans lequel le groupe alkylène est optionnellement interrompu par un ou plusieurs atomes d'oxygène.

11. Utilisation selon la revendication 10, dans laquelle le composé est tel que défini dans l'une quelconque des revendications 2 à 4.

12. Utilisation d'un composé selon l'une quelconque des revendications 1 à 4 pour la préparation d'une composition pharmaceutique destinée à améliorer l'apprentissage et la mémoire.

13. Utilisation de cellules souches différenciées susceptibles être obtenues par le procédé selon la revendication 6 ou 7 pour la préparation d'une composition pharmaceutique destinée au traitement d'un trouble neuronal.
